# EUROPEAN PATENT APPLICATION

(11) **EP 1 473 368 A1**
(43) Date of publication of application: **03.11.2004**
(21) Application number: 03706925.9
(22) Date of filing: 06.02.2003
(51) Int. Cl.: C12N 15/53, C12N 9/02, C12N 5/10, C12N 1/15, C12N 1/19, C12N 1/21, C12P 7/40

(54) **ALPHA-SUBSTITUTED-ALPHA, BETA-UNSATURATED CARBONYL COMPOUND REDUCTASE GENE**

(30) Priority: 06.02.2002 JP 2002030127; 26.09.2002 JP 2002281236
(71) Applicant: SHOWA DENKO K.K., Tokyo 105-8518 (JP)
(72) Inventor: KAMACHI, Harumi, Corporate R & D Center, Chiba-shi, Chiba 267-0056 (JP); ESAKI, Nobuyoshi, Otsu-shi, Shiga 520-2279 (JP); KURIHARA, Tatsuo, Fushimi Godo Shukusha 533, Kyoto-shi, Kyoto 612-8104 (JP)
(74) Representative: Strehl Schübel-Hopf & Partner
(86) International application number: PCT/JP2003/001240
(87) International publication number: WO 2003/066863

(57) **Abstract**

The present invention relates to: a reductase gene for an α-substituted-α,β-unsaturated carbonyl compound which contains a DNA sequence encoding an amino acid sequence represented by SEQ ID NO: 20 and an amino acid sequence represented by SEQ ID NO: 21; an enzyme which is a product of the gene; a plasmid and a transformant each containing the gene DNA; and a method of reducing an α-substituted-α,β-unsaturated carbonyl compound using the transformant. According to claim the present invention, there is provided an enzyme gene which is useful in producing a corresponding α-substituted-α,β-saturated carbonyl compound optically active at the α-position by hydrogenating an α,β-carbon double bond of an α-substituted carbonyl compound, which is a compound prochiral at the alpha-position, and an enzyme which is a gene product thereof.

SEQUENCE LISTING

<110>  SHOWA DENKO K.K.

<120>  REDUCTASE GENE FOR α-SUBSTITUTED-α,β-UNSATURATED CARBONYL COMPOUND

<130>  EPA-63516

<150>  JP2002-030127
<151>  2002-02-06

<150>  JP2002-281236
<151>  2002-09-26

<160>  23

<170>  PatentIn version 3.1

<210>  1
<211>  16
<212>  PRT
<213>  Burkholderia sp.

<400>  1

Val Met Ala Ala Val Ile His Lys Lys Gly Gly Pro Asp Asn Phe Val
1               5                   10                  15

<210>  2
<211>  15
<212>  PRT
<213>  Burkholderia sp.

<400>  2

Asp Leu Asp Leu Asp Asp Val His Leu Ala Gly Leu Met Leu Lys
1               5                   10                  15

<210> 3
<211> 13
<212> PRT
<213> Burkholderia sp.

<220>
<221> MISC_FEATURE
<222> (1)..(1)
<223> Unknown

<220>
<221> MISC_FEATURE
<222> (12)..(12)
<223> Unknown

<400> 3

Xaa Asp Val Leu Val Thr Asp Val Leu Val Val Xaa Gly
1               5                   10

<210> 4
<211> 25
<212> PRT
<213> Burkholderia sp.

<400> 4

Val Phe Val Asp Phe Arg Glu Thr Lys Pro Glu Glu Trp Ala Pro Asp
1               5                   10                  15

Ser Leu Thr Gly Thr Phe Leu Gly Lys
            20                  25

<210> 5
<211> 30
<212> PRT

<213>  Burkholderia sp.

<400>  5

Glu Leu Leu Ser Gly Leu Asp Ala Asp Tyr Gly Thr Arg Gly Ser Leu
1               5                   10                  15

Glu Asp Thr Thr Gly Leu Met Met Glu Phe Ser Ser Thr His
            20                  25                  30

<210>  6
<211>  20
<212>  DNA
<213>  Artificial

<220>
<223>  Forword primer for N-terminal fragment of CAA43

<220>
<221>  misc_feature
<222>  (6)..(6)
<223>  Inosine

<220>
<221>  misc_feature
<222>  (9)..(9)
<223>  Inosine

<220>
<221>  misc_feature
<222>  (12)..(12)
<223>  Inosine

<400>  6
atggcngcng tnathcayaa                                                20

<210>  7

<211>  20
<212>  DNA
<213>  Artificial

<220>
<223>  Reverse primer for N-terminal fragment of CAA43

<220>
<221>  misc_feature
<222>  (3)..(3)
<223>  Inosine

<220>
<221>  misc_feature
<222>  (6)..(6)
<223>  Inosine

<220>
<221>  misc_feature
<222>  (12)..(12)
<223>  Inosine

<400>  7
ccngcnarrt gnacrtcgtc                                                    20

<210>  8
<211>  20
<212>  DNA
<213>  Artificial

<220>
<223>  Forword primer for C-terminal fragment of CAA43

<400>  8
cgcccctcgg tgcctacagc                                                    20

<210>  9

<211> 20

<212> DNA

<213> Artificial

<220>

<223> Reverse primer for C-terminal fragment of CAA43

<400> 9
   ctaccccgcc gaaaaactga 20

<210> 10

<211> 20

<212> DNA

<213> Artificial

<220>

<223> Primer for inverted PCR

<400> 10
ccgggcgagc caaccttaac 20

<210> 11

<211> 350

<212> DNA

<213> Burkholderia sp.

<400> 11
ggtaattcat aagaagggtg gacccgataa cttcgtatgg gaggaagtta aggttggctc 60

gcccggcccg ggtcaagtgc gactgcgcaa tacggccatc ggggtaaact tcctggatac 120

ttatcaccgc gcaggcattc ctcacccgct ggtcgttggc gagccgccga ttgtggtcgg 180

cttcgaagcc gccgctgtgg ttgaggaagt cggtcccggt gtaaccgact tcaccgttgg 240

tgagcgggtc tgcacttgtc ttccgcccct cggtgcctac agccaggagc gcctctaccc 300

cgccgaaaaa ctgatcaagg ttccaaagga cctggatctt gatgacgtgc 350

<210> 12
<211> 1069
<212> DNA
<213> Burkholderia sp.

<400> 12
tcaaggttcc aaaggacctg gatcttgatg acgtgcacct cgcgggattg atgctcaagg 60

ggatgacagc acaatatctg ctgcatcaga cgcacaaggt aaagccgggt gactacgtgt 120

tgattcacgc ggcggctggc ggcatgggcc acatcatggt tccttgggcg cgccacctcg 180

gcgctaccgt gatcgggacg gtcagcacgg aagaaaaggc tgagactgct cgcaaactcg 240

gctgccacca taccatcaat tattccactc aggatttcgc tgaggtagtt cgcgaaatca 300

cgggcgggaa gggtgtcgac gtggtctacg attccatcgg taaagacaca ctccagaagt 360

cgctcgactg tctgcggccg cgcggtatgt gtgcggccta cgggcacgca tccggcgtgg 420

cagatccgat cagggtcgtc gaggacctcg gtgtacgtgg atcgctgttc attactagac 480

ccgcactctg gcattacatg tcgaaccgca gtgagattga cgaagggtcg aagtgcctgt 540

tcgatgccgt caaggcgggt gtactccata gcagtgtcgc aaagaccttc cctctgcggg 600

aggcagcggc ggcgcacaaa tacatgggtg gtcgtcagac gatcggctcg attgttttgc 660

ttccgcaagc gtaggtagcc gtagggcgtc accccggaat ttcggggtga ccgaaaacgt 720

cgccgtgaac gcctatctgc ggatgttggc ggttgggtgg cattattttt cggcagcccg 780

tcgttcgctg ccggctcgta cgttgccggt ttggacagtt cgtcaacggg gcgattgtga 840

tttccaaccc gcgaccctcg attgggtcaa cgtgtgtttg actttctgtg aactctgtct 900

aagtacacga gaggtgtgta gatcagacgc gtgccgaagc tactcggatg ttccctgtcg 960

aggcaattga aagagtcaat acacatgaac gtattcaata gagagatagt gctttccgtc 1020

cggcactgga cggataaact tttcagcttt accgcaaccc gtaacgccg 1069

<210> 13
<211> 1617
<212> DNA
<213> Burkholderia sp.

<400> 13
gtcgacttcc gcgaaacgaa accggaggaa tgggcgcctg attcactcac aggcaccttc 60

ttgggcaagt gtgtcccgaa tttcatgacc accccggtac aggttgcgcc gtcatcgcac 120

tacacgatcg gcggtctgaa agtcgatgtg gatggccgta ccaatcttcc gaagctctac 180

gctgtcggcg agttggccgg tggcgtgcat ggcgccaacc gccacggtgg cacggcgctg 240

gtcgatgcca tgacgtacgg ccggattgct ggacggcacg cggcgggaag cctcaacggc 300

gcggctgcga cgggaggtgc agcgcttcta cccgcaggca gcaaagcggg aaaggcaagc 360

cggattgagg gcgcaatgag cgatctgcgc cgcgcaaacc agctcgctct tggtcctatt 420

cgtgatgccg tacggcttca acgcgttggg gagctgtttg ctgaactctt ggacgaggtc 480

cgctcgttcg gttggaacgg ctacaaggaa atgcaggaaa tcttgcgcgt cgagcgtgcg 540

atcaagctgt ctgacgctat gcgccaggcc atgttacgcc gcacggagac acgcggagtc 600

cactatcggg ccgatttccc gagctccagt gatgcatggt tgaagaagca ggtatttgca 660

ttgcgcgatg ggcgttgca tttcaaagac gttcccctct aatcaaagtc gcataagcgc 720

gatattgcat caggtatttg tctcgctgtg gtgtcatttg tgcctcggcg cccaagggtg 780

agagcggaag gtggagctcg ccgccgcggg cagatctggt gtgtgtcgtt gttccgtatc 840

ggtagcaaaa acaatctgac ttcgctagcg ggcaggtaag ccgacggcgt atgctcgccg 900

aaggttcttg aattgagttc gtggtgacca tgtcgctgcg gtgtgaatcg ctatttagga 960

gactttatca tggtaatggc agcggtaatt cataagaagg gtggacccga taacttcgta 1020

tgggaggaag ttaaggttgg ctcgcccggc ccgggtcaag tgcgactgcg caatacggcc 1080

atcggggtaa acttcctgga tacttatcac cgcgcaggca ttcctcaccc gctggtcgtt 1140

ggcgagccgc cgattgtggt cggcttcgaa gccgccgctg tggttgagga agtcggtccc 1200

ggtgtaaccg acttcaccgt tggtgagcgg gtctgcactt gtcttccgcc cctcggtgcc 1260

tacagccagg agcgcctcta ccccgccgaa aaactgatca aggttccaaa ggacctggat 1320

cttgatgacg tgcacctcgc gggattgatg ctcaagggga tgacagcaca atatctgctg 1380

catcagacgc acaaggtaaa gccgggtgac tacgtgttga ttcacgcggc ggctggcggc 1440

atgggccaca tcatggttcc ttgggcgcgc cacctcggcg ctaccgtgat cgggacggtc 1500

agcacggaag aaaaggctga gactgctcgc aaactcggct gccaccatac catcaattat 1560

tccactcagg atttcgctga ggtagttcgc gaaatcacgg gcgggaaggg tgtcgac 1617

<210> 14
<211> 20
<212> DNA
<213> Artificial

<220>
<223> Forword primer for N-terminal fragment of CAA67

<400> 14
catgaaattc gggacacact 20

<210> 15
<211> 20
<212> DNA
<213> Artificial

<220>
<223> Reverse primer for N-terminal fragment of CAA67

<400> 15
agaaggtgcc tgtgagtgaa 20

<210> 16
<211> 1640
<212> DNA
<213> Burkholderia sp.

<400> 16
aagcgtactc cagcgttagc tggccaatcc ttgcgtggag caccgtgacg tccactggtg 60

gctcaaacgt cgttgacttg cctgtctcga acacttccga cacgcgctcc tgcaactgct 120

tcttccagtc tgtaatctgg ttcggatgaa cgtcgcactg ctgcgccagt tctgccagcg 180

tcttgtcgcc cctcggggct gccagcccta tctttgcctt gaataccgtg ctatgcgacc 240

ggcggcttct tttgtcatcg ttgggctcct cttgccggtc cttataccag ttcagttccc 300

ggccgttcga cttaccgacc tgcctgaatt tccggccgca cctctaaccc aagatcattt 360

ctgacgattt ggtagaagtt ttctgcgttc aggccacttt ttcggccgct ttccgagggg 420

atagtattgc gacaaatgtg agcgatccgt agccaacggg tattgcgagg cggctgccgc 480

ttcggcgggt cacacctatc ctgtgtggtc gcacacaagg ttcgcgacgt caataaagat 540

gatttggaga catgcacgtg atgttctccc ttgatgtcta gcggtcgttg aggatcattt 600

aatccaatgt ttgacaggag gaggatgttc atgtcggatg ttcttgtaac agacgtgttg 660

gtggtgggcg agggctgcgc aggccaaacc gctgcgctta ctgcaagcga gtcgggttgt 720

gacgtcatca tgcttggaga cggccgcgca ccgagcaccg ctgtttccac cggcttcctt 780

acttatgccg cgcacgaagg tttcaatcgt gcccagctct acgaagcgat gtcacagacc 840

acaggcaagg gcttgtgtga tgtagcgctc ttgaggcgac ttgtcgatga agctccgaaa 900

gaaatggcgg agttgattga gacatataag gttcctgtcg ataacaccga gcgtggagtg 960

agggcgcgcc gggcagtggg taagagcgga aaagagcttc tctccggatt ggacgcggat 1020

tacgggacgc gtggttccct cgaggacacg acgggcctaa tgatggagtt ctcgtcgaca 1080

cacggaacag cgctctatgc ccagttgcgt aaagccgtga acacggcgcc aaagattcgg 1140

cgcgtacgcg gaagtgcgct ggttctcgaa cccggttcca ccacggtcgg tgcgcttgtc 1200

gatggcgagc cggtgacgat cgcggctcgt tcgatcatct tggcgactgg agggattcag 1260

ggcctctacg aggtcacgga taacccgcat acgctcacgg gtgatggtca tggcatggcg 1320

atggacgctg gcgcggagtt cgtcgacatg gagttcatgc agttctaccc gctttcagtc 1380

aatgaggagg gcgcaccgac actcttcttc tatccggatt tccccaggcg cgccaagctc 1440

attgacgacg gggccgaaa cgtcctggtg aagcatctcg gcgagggctc gcaatacctt 1500

tcggagttgc ataattggga tcagctagcc gcggtggtac agacggagat tgtcgaaggc 1560

aggaaggtat ttgtcgactt ccgcgaaacg aaaccggagg aatgggcgcc tgattcactc 1620

acaggcacct tcttgggcaa                                                    1640

<210>  17
<211>  1002
<212>  DNA
<213>  Burkholderia sp.

<220>
<221>  CDS
<222>  (1)..(1002)
<223>

<400>  17
atg gta atg gca gcg gta att cat aag aag ggt gga ccc gat aac ttc      48
Met Val Met Ala Ala Val Ile His Lys Lys Gly Gly Pro Asp Asn Phe
1               5                   10                  15

gta tgg gag gaa gtt aag gtt ggc tcg ccc ggc ccg ggt caa gtg cga      96
Val Trp Glu Glu Val Lys Val Gly Ser Pro Gly Pro Gly Gln Val Arg
            20                  25                  30

ctg cgc aat acg gcc atc ggg gta aac ttc ctg gat act tat cac cgc      144
Leu Arg Asn Thr Ala Ile Gly Val Asn Phe Leu Asp Thr Tyr His Arg
        35                  40                  45

gca ggc att cct cac ccg ctg gtc gtt ggc gag ccg ccg att gtg gtc      192
Ala Gly Ile Pro His Pro Leu Val Val Gly Glu Pro Pro Ile Val Val
    50                  55                  60

ggc ttc gaa gcc gcc gct gtg gtt gag gaa gtc ggt ccc ggt gta acc      240
Gly Phe Glu Ala Ala Ala Val Val Glu Glu Val Gly Pro Gly Val Thr
65                  70                  75                  80

gac ttc acc gtt ggt gag cgg gtc tgc act tgt ctt ccg ccc ctc ggt      288
Asp Phe Thr Val Gly Glu Arg Val Cys Thr Cys Leu Pro Pro Leu Gly
                85                  90                  95

gcc tac agc cag gag cgc ctc tac ccc gcc gaa aaa ctg atc aag gtt 336
Ala Tyr Ser Gln Glu Arg Leu Tyr Pro Ala Glu Lys Leu Ile Lys Val
            100                 105                 110

cca aag gac ctg gat ctt gat gac gtg cac ctc gcg gga ttg atg ctc 384
Pro Lys Asp Leu Asp Leu Asp Asp Val His Leu Ala Gly Leu Met Leu
        115                 120                 125

aag ggg atg aca gca caa tat ctg ctg cat cag acg cac aag gta aag 432
Lys Gly Met Thr Ala Gln Tyr Leu Leu His Gln Thr His Lys Val Lys
    130                 135                 140

ccg ggt gac tac gtg ttg att cac gcg gcg gct ggc ggc atg ggc cac 480
Pro Gly Asp Tyr Val Leu Ile His Ala Ala Ala Gly Gly Met Gly His
145                 150                 155                 160

atc atg gtt cct tgg gcg cgc cac ctc ggc gct acc gtg atc ggg acg 528
Ile Met Val Pro Trp Ala Arg His Leu Gly Ala Thr Val Ile Gly Thr
                165                 170                 175

gtc agc acg gaa gaa aag gct gag act gct cgc aaa ctc ggc tgc cac 576
Val Ser Thr Glu Glu Lys Ala Glu Thr Ala Arg Lys Leu Gly Cys His
            180                 185                 190

cat acc atc aat tat tcc act cag gat ttc gct gag gta gtt cgc gaa 624
His Thr Ile Asn Tyr Ser Thr Gln Asp Phe Ala Glu Val Val Arg Glu
        195                 200                 205

atc acg ggc ggg aag ggt gtc gac gtg gtc tac gat tcc atc ggt aaa 672
Ile Thr Gly Gly Lys Gly Val Asp Val Val Tyr Asp Ser Ile Gly Lys
    210                 215                 220

gac aca ctc cag aag tcg ctc gac tgt ctg cgg ccg cgc ggt atg tgt 720
Asp Thr Leu Gln Lys Ser Leu Asp Cys Leu Arg Pro Arg Gly Met Cys
225                 230                 235                 240

gcg gcc tac ggg cac gca tcc ggc gtg gca gat ccg atc agg gtc gtc     768
Ala Ala Tyr Gly His Ala Ser Gly Val Ala Asp Pro Ile Arg Val Val
                245                 250                 255

gag gac ctc ggt gta cgt gga tcg ctg ttc att act aga ccc gca ctc     816
Glu Asp Leu Gly Val Arg Gly Ser Leu Phe Ile Thr Arg Pro Ala Leu
            260                 265                 270

tgg cat tac atg tcg aac cgc agt gag att gac gaa ggg tcg aag tgc     864
Trp His Tyr Met Ser Asn Arg Ser Glu Ile Asp Glu Gly Ser Lys Cys
        275                 280                 285

ctg ttc gat gcc gtc aag gcg ggt gta ctc cat agc agt gtc gca aag     912
Leu Phe Asp Ala Val Lys Ala Gly Val Leu His Ser Ser Val Ala Lys
    290                 295                 300

acc ttc cct ctg cgg gag gca gcg gcg gcg cac aaa tac atg ggt ggt     960
Thr Phe Pro Leu Arg Glu Ala Ala Ala Ala His Lys Tyr Met Gly Gly
305                 310                 315                 320

cgt cag acg atc ggc tcg att gtt ttg ctt ccg caa gcg tag             1002
Arg Gln Thr Ile Gly Ser Ile Val Leu Leu Pro Gln Ala
                325                 330

<210>  18
<211>  1644
<212>  DNA
<213>  Burkholderia sp.

<220>
<221>  CDS
<222>  (1)..(1644)
<223>

<400>  18
atg tcg gat gtt ctt gta aca gac gtg ttg gtg gtg ggc gag ggc tgc     48
Met Ser Asp Val Leu Val Thr Asp Val Leu Val Val Gly Glu Gly Cys

1                    5                        10                       15

gca ggc caa acc gct gcg ctt act gca agc gag tcg ggt tgt gac gtc          96
Ala Gly Gln Thr Ala Ala Leu Thr Ala Ser Glu Ser Gly Cys Asp Val
                20                       25                       30

atc atg ctt gga gac ggc cgc gca ccg agc acc gct gtt tcc acc ggc          144
Ile Met Leu Gly Asp Gly Arg Ala Pro Ser Thr Ala Val Ser Thr Gly
            35                       40                       45

ttc ctt act tat gcc gcg cac gaa ggt ttc aat cgt gcc cag ctc tac          192
Phe Leu Thr Tyr Ala Ala His Glu Gly Phe Asn Arg Ala Gln Leu Tyr
    50                       55                       60

gaa gcg atg tca cag acc aca ggc aag ggc ttg tgt gat gta gcg ctc          240
Glu Ala Met Ser Gln Thr Thr Gly Lys Gly Leu Cys Asp Val Ala Leu
65                       70                       75                       80

ttg agg cga ctt gtc gat gaa gct ccg aaa gaa atg gcg gag ttg att          288
Leu Arg Arg Leu Val Asp Glu Ala Pro Lys Glu Met Ala Glu Leu Ile
                    85                       90                       95

gag aca tat aag gtt cct gtc gat aac acc gag cgt gga gtg agg gcg          336
Glu Thr Tyr Lys Val Pro Val Asp Asn Thr Glu Arg Gly Val Arg Ala
                100                      105                      110

cgc cgg gca gtg ggt aag agc gga aaa gag ctt ctc tcc gga ttg gac          384
Arg Arg Ala Val Gly Lys Ser Gly Lys Glu Leu Leu Ser Gly Leu Asp
            115                      120                      125

gcg gat tac ggg acg cgt ggt tcc ctc gag gac acg acg ggc cta atg          432
Ala Asp Tyr Gly Thr Arg Gly Ser Leu Glu Asp Thr Thr Gly Leu Met
    130                      135                      140

atg gag ttc tcg tcg aca cac gga aca gcg ctc tat gcc cag ttg cgt          480
Met Glu Phe Ser Ser Thr His Gly Thr Ala Leu Tyr Ala Gln Leu Arg
145                      150                      155                      160

aaa gcc gtg aac acg gcg cca aag att cgg cgc gta cgc gga agt gcg 528
Lys Ala Val Asn Thr Ala Pro Lys Ile Arg Arg Val Arg Gly Ser Ala
                165                 170                 175

ctg gtt ctc gaa ccc ggt tcc acc acg gtc ggt gcg ctt gtc gat ggc 576
Leu Val Leu Glu Pro Gly Ser Thr Thr Val Gly Ala Leu Val Asp Gly
            180                 185                 190

gag ccg gtg acg atc gcg gct cgt tcg atc atc ttg gcg act gga ggg 624
Glu Pro Val Thr Ile Ala Ala Arg Ser Ile Ile Leu Ala Thr Gly Gly
        195                 200                 205

att cag ggc ctc tac gag gtc acg gat aac ccg cat acg ctc acg ggt 672
Ile Gln Gly Leu Tyr Glu Val Thr Asp Asn Pro His Thr Leu Thr Gly
    210                 215                 220

gat ggt cat ggc atg gcg atg gac gct ggc gcg gag ttc gtc gac atg 720
Asp Gly His Gly Met Ala Met Asp Ala Gly Ala Glu Phe Val Asp Met
225                 230                 235                 240

gag ttc atg cag ttc tac ccg ctt tca gtc aat gag gag ggc gca ccg 768
Glu Phe Met Gln Phe Tyr Pro Leu Ser Val Asn Glu Glu Gly Ala Pro
                245                 250                 255

aca ctc ttc ttc tat ccg gat ttc ccc agg cgc gcc aag ctc att gac 816
Thr Leu Phe Phe Tyr Pro Asp Phe Pro Arg Arg Ala Lys Leu Ile Asp
            260                 265                 270

gac ggg ggc cga aac gtc ctg gtg aag cat ctc ggc gag ggc tcg caa 864
Asp Gly Gly Arg Asn Val Leu Val Lys His Leu Gly Glu Gly Ser Gln
        275                 280                 285

tac ctt tcg gag ttg cat aat tgg gat cag cta gcc gcg gtg gta cag 912
Tyr Leu Ser Glu Leu His Asn Trp Asp Gln Leu Ala Ala Val Val Gln
    290                 295                 300

acg gag att gtc gaa ggc agg aag gta ttt gtc gac ttc cgc gaa acg 960
Thr Glu Ile Val Glu Gly Arg Lys Val Phe Val Asp Phe Arg Glu Thr
305                 310                 315                 320

aaa ccg gag gaa tgg gcg cct gat tca ctc aca ggc acc ttc ttg ggc 1008
Lys Pro Glu Glu Trp Ala Pro Asp Ser Leu Thr Gly Thr Phe Leu Gly
                325                 330                 335

aag tgt gtc ccg aat ttc atg acc acc ccg gta cag gtt gcg ccg tca 1056
Lys Cys Val Pro Asn Phe Met Thr Thr Pro Val Gln Val Ala Pro Ser
            340                 345                 350

tcg cac tac acg atc ggc ggt ctg aaa gtc gat gtg gat ggc cgt acc 1104
Ser His Tyr Thr Ile Gly Gly Leu Lys Val Asp Val Asp Gly Arg Thr
        355                 360                 365

aat ctt ccg aag ctc tac gct gtc ggc gag ttg gcc ggt ggc gtg cat 1152
Asn Leu Pro Lys Leu Tyr Ala Val Gly Glu Leu Ala Gly Gly Val His
    370                 375                 380

ggc gcc aac cgc cac ggt ggc acg gcg ctg gtc gat gcc atg acg tac 1200
Gly Ala Asn Arg His Gly Gly Thr Ala Leu Val Asp Ala Met Thr Tyr
385                 390                 395                 400

ggc cgg att gct gga cgg cac gcg gcg gga agc ctc aac ggc gcg gct 1248
Gly Arg Ile Ala Gly Arg His Ala Ala Gly Ser Leu Asn Gly Ala Ala
                405                 410                 415

gcg acg gga ggt gca gcg ctt cta ccc gca ggc agc aaa gcg gga aag 1296
Ala Thr Gly Gly Ala Ala Leu Leu Pro Ala Gly Ser Lys Ala Gly Lys
            420                 425                 430

gca agc cgg att gag ggc gca atg agc gat ctg cgc cgc gca aac cag 1344
Ala Ser Arg Ile Glu Gly Ala Met Ser Asp Leu Arg Arg Ala Asn Gln
        435                 440                 445

ctc gct ctt ggt cct att cgt gat gcc gta cgg ctt caa cgc gtt ggg 1392

Leu Ala Leu Gly Pro Ile Arg Asp Ala Val Arg Leu Gln Arg Val Gly
450                 455                 460

gag ctg ttt gct gaa ctc ttg gac gag gtc cgc tcg ttc ggt tgg aac     1440
Glu Leu Phe Ala Glu Leu Leu Asp Glu Val Arg Ser Phe Gly Trp Asn
465                 470                 475                 480

ggc tac aag gaa atg cag gaa atc ttg cgc gtc gag cgt gcg atc aag     1488
Gly Tyr Lys Glu Met Gln Glu Ile Leu Arg Val Glu Arg Ala Ile Lys
                485                 490                 495

ctg tct gac gct atg cgc cag gcc atg tta cgc cgc acg gag aca cgc     1536
Leu Ser Asp Ala Met Arg Gln Ala Met Leu Arg Arg Thr Glu Thr Arg
            500                 505                 510

gga gtc cac tat cgg gcc gat ttc ccg agc tcc agt gat gca tgg ttg     1584
Gly Val His Tyr Arg Ala Asp Phe Pro Ser Ser Ser Asp Ala Trp Leu
        515                 520                 525

aag aag cag gta ttt gca ttg cgc gat ggg gcg ttg cat ttc aaa gac     1632
Lys Lys Gln Val Phe Ala Leu Arg Asp Gly Ala Leu His Phe Lys Asp
    530                 535                 540

gtt ccc ctc taa                                                     1644
Val Pro Leu
545

<210>  19
<211>  3938
<212>  DNA
<213>  Burkholderia sp.

<400>  19
aagcgtactc cagcgttagc tggccaatcc ttgcgtggag caccgtgacg tccactggtg      60

gctcaaacgt cgttgacttg cctgtctcga acacttccga cacgcgctcc tgcaactgct     120

tcttccagtc tgtaatctgg ttcggatgaa cgtcgcactg ctgcgccagt tctgccagcg 180

tcttgtcgcc cctcggggct gccagcccta tctttgcctt gaataccgtg ctatgcgacc 240

ggcggcttct tttgtcatcg ttgggctcct cttgccggtc cttataccag ttcagttccc 300

ggccgttcga cttaccgacc tgcctgaatt tccggccgca cctctaaccc aagatcattt 360

ctgacgattt ggtagaagtt ttctgcgttc aggccacttt ttcggccgct ttccgagggg 420

atagtattgc gacaaatgtg agcgatccgt agccaacggg tattgcgagg cggctgccgc 480

ttcggcgggt cacacctatc ctgtgtggtc gcacacaagg ttcgcgacgt caataaagat 540

gatttggaga catgcacgtg atgttctccc ttgatgtcta gcggtcgttg aggatcattt 600

aatccaatgt ttgacaggag gaggatgttc atgtcggatg ttcttgtaac agacgtgttg 660

gtggtgggcg agggctgcgc aggccaaacc gctgcgctta ctgcaagcga gtcgggttgt 720

gacgtcatca tgcttggaga cggccgcgca ccgagcaccg ctgtttccac cggcttcctt 780

acttatgccg cgcacgaagg tttcaatcgt gcccagctct acgaagcgat gtcacagacc 840

acaggcaagg gcttgtgtga tgtagcgctc ttgaggcgac ttgtcgatga agctccgaaa 900

gaaatggcgg agttgattga gacatataag gttcctgtcg ataacaccga gcgtggagtg 960

agggcgcgcc gggcagtggg taagagcgga aaagagcttc tctccggatt ggacgcggat 1020

tacgggacgc gtggttccct cgaggacacg acgggcctaa tgatggagtt ctcgtcgaca 1080

cacggaacag cgctctatgc ccagttgcgt aaagccgtga acacggcgcc aaagattcgg 1140

cgcgtacgcg gaagtgcgct ggttctcgaa cccggttcca ccacggtcgg tgcgcttgtc 1200

gatggcgagc cggtgacgat cgcggctcgt tcgatcatct tggcgactgg agggattcag 1260

ggcctctacg aggtcacgga taacccgcat acgctcacgg gtgatggtca tggcatggcg 1320

atggacgctg gcgcggagtt cgtcgacatg gagttcatgc agttctaccc gctttcagtc 1380

aatgaggagg gcgcaccgac actcttcttc tatccggatt tccccaggcg cgccaagctc 1440

attgacgacg ggggccgaaa cgtcctggtg aagcatctcg gcgagggctc gcaatacctt 1500

tcggagttgc ataattggga tcagctagcc gcggtggtac agacggagat tgtcgaaggc 1560

aggaaggtat ttgtcgactt ccgcgaaacg aaaccggagg aatgggcgcc tgattcactc 1620

acaggcacct tcttgggcaa gtgtgtcccg aatttcatga ccaccccggt acaggttgcg 1680

ccgtcatcgc actacacgat cggcggtctg aaagtcgatg tggatggccg taccaatctt 1740

ccgaagctct acgctgtcgg cgagttggcc ggtggcgtgc atggcgccaa ccgccacggt 1800

ggcacggcgc tggtcgatgc catgacgtac ggccggattg ctggacggca cgcggcggga 1860

agcctcaacg gcgcggctgc gacgggaggt gcagcgcttc tacccgcagg cagcaaagcg 1920

ggaaaggcaa gccggattga gggcgcaatg agcgatctgc gccgcgcaaa ccagctcgct 1980

cttggtccta ttcgtgatgc cgtacggctt caacgcgttg ggagctgttt tgctgaactc 2040

ttggacgagg tccgctcgtt cggttggaac ggctacaagg aaatgcagga aatcttgcgc 2100

gtcgagcgtg cgatcaagct gtctgacgct atgcgccagg ccatgttacg ccgcacggag 2160

acacgcggag tccactatcg ggccgatttc ccgagctcca gtgatgcatg gttgaagaag 2220

caggtatttg cattgcgcga tggggcgttg catttcaaag acgttcccct ctaatcaaag 2280

tcgcataagc gcgatattgc atcaggtatt tgtctcgctg tggtgtcatt tgtgcctcgg 2340

cgcccaaggg tgagagcgga aggtggagct cgccgccgcg ggcagatctg gtgtgtgtcg 2400

ttgttccgta tcggtagcaa aaacaatctg acttcgctag cgggcaggta agccgacggc 2460

gtatgctcgc cgaaggttct tgaattgagt tcgtggtgac catgtcgctg cggtgtgaat 2520

cgctatttag gagactttat catggtaatg gcagcggtaa ttcataagaa gggtggaccc 2580

gataacttcg tatgggagga agttaaggtt ggctcgcccg gcccgggtca agtgcgactg 2640

cgcaatacgg ccatcggggt aaacttcctg gatacttatc accgcgcagg cattcctcac 2700

ccgctggtcg ttggcgagcc gccgattgtg gtcggcttcg aagccgccgc tgtggttgag 2760

gaagtcggtc ccggtgtaac cgacttcacc gttggtgagc gggtctgcac ttgtcttccg 2820

cccctcggtg cctacagcca ggagcgcctc taccccgccg aaaaactgat caaggttcca 2880

aaggacctgg atcttgatga cgtgcacctc gcgggattga tgctcaaggg gatgacagca 2940

caatatctgc tgcatcagac gcacaaggta aagccgggtg actacgtgtt gattcacgcg 3000

gcggctggcg gcatgggcca catcatggtt ccttgggcgc gccacctcgg cgctaccgtg 3060

atcgggacgg tcagcacgga agaaaaggct gagactgctc gcaaactcgg ctgccaccat 3120

accatcaatt attccactca ggatttcgct gaggtagttc gcgaaatcac gggcgggaag 3180

ggtgtcgacg tggtctacga ttccatcggt aaagacacac tccagaagtc gctcgactgt 3240

ctgcggccgc gcggtatgtg tgcggcctac gggcacgcat ccggcgtggc agatccgatc 3300

agggtcgtcg aggacctcgg tgtacgtgga tcgctgttca ttactagacc cgcactctgg 3360

cattacatgt cgaaccgcag tgagattgac gaagggtcga agtgcctgtt cgatgccgtc 3420

aaggcgggtg tactccatag cagtgtcgca aagaccttcc ctctgcggga ggcagcggcg 3480

gcgcacaaat acatgggtgg tcgtcagacg atcggctcga ttgttttgct tccgcaagcg 3540

taggtagccg tagggcgtca ccccggaatt tcggggtgac cgaaaacgtc gccgtgaacg 3600

cctatctgcg gatgttggcg gttgggtggc attatttttc ggcagcccgt cgttcgctgc 3660

cggctcgtac gttgccggtt tggacagttc gtcaacgggg cgattgtgat ttccaacccg 3720

cgaccctcga ttgggtcaac gtgtgtttga ctttctgtga actctgtcta agtacacgag 3780

aggtgtgtag atcagacgcg tgccgaagct actcggatgt tccctgtcga ggcaattgaa 3840

agagtcaata cacatgaacg tattcaatag agagatagtg ctttccgtcc ggcactggac 3900

ggataaactt ttcagcttta ccgcaacccg taacgccg 3938

<210> 20
<211> 333
<212> PRT
<213> Burkholderia sp.

<400> 20

Met Val Met Ala Ala Val Ile His Lys Lys Gly Gly Pro Asp Asn Phe
1               5                   10                  15

Val Trp Glu Glu Val Lys Val Gly Ser Pro Gly Pro Gly Gln Val Arg
            20                  25                  30

Leu Arg Asn Thr Ala Ile Gly Val Asn Phe Leu Asp Thr Tyr His Arg
        35                  40                  45

Ala Gly Ile Pro His Pro Leu Val Val Gly Glu Pro Pro Ile Val Val
    50                  55                  60

Gly Phe Glu Ala Ala Ala Val Val Glu Glu Val Gly Pro Gly Val Thr

65                  70                  75                  80

Asp Phe Thr Val Gly Glu Arg Val Cys Thr Cys Leu Pro Pro Leu Gly
                85                  90                  95

Ala Tyr Ser Gln Glu Arg Leu Tyr Pro Ala Glu Lys Leu Ile Lys Val
            100                 105                 110

Pro Lys Asp Leu Asp Leu Asp Asp Val His Leu Ala Gly Leu Met Leu
        115                 120                 125

Lys Gly Met Thr Ala Gln Tyr Leu Leu His Gln Thr His Lys Val Lys
    130                 135                 140

Pro Gly Asp Tyr Val Leu Ile His Ala Ala Ala Gly Gly Met Gly His
145                 150                 155                 160

Ile Met Val Pro Trp Ala Arg His Leu Gly Ala Thr Val Ile Gly Thr
                165                 170                 175

Val Ser Thr Glu Glu Lys Ala Glu Thr Ala Arg Lys Leu Gly Cys His
            180                 185                 190

His Thr Ile Asn Tyr Ser Thr Gln Asp Phe Ala Glu Val Val Arg Glu
        195                 200                 205

Ile Thr Gly Gly Lys Gly Val Asp Val Val Tyr Asp Ser Ile Gly Lys
    210                 215                 220

Asp Thr Leu Gln Lys Ser Leu Asp Cys Leu Arg Pro Arg Gly Met Cys
225                 230                 235                 240

Ala Ala Tyr Gly His Ala Ser Gly Val Ala Asp Pro Ile Arg Val Val
                245                 250                 255

Glu Asp Leu Gly Val Arg Gly Ser Leu Phe Ile Thr Arg Pro Ala Leu
            260                 265                 270

Trp His Tyr Met Ser Asn Arg Ser Glu Ile Asp Glu Gly Ser Lys Cys
        275                 280                 285

Leu Phe Asp Ala Val Lys Ala Gly Val Leu His Ser Ser Val Ala Lys
    290                 295                 300

Thr Phe Pro Leu Arg Glu Ala Ala Ala Ala His Lys Tyr Met Gly Gly
305                 310                 315                 320

Arg Gln Thr Ile Gly Ser Ile Val Leu Leu Pro Gln Ala
                325                 330

<210> 21
<211> 547
<212> PRT
<213> Burkholderia sp.

<400> 21

Met Ser Asp Val Leu Val Thr Asp Val Leu Val Val Gly Glu Gly Cys
1               5                   10                  15

Ala Gly Gln Thr Ala Ala Leu Thr Ala Ser Glu Ser Gly Cys Asp Val
            20                  25                  30

Ile Met Leu Gly Asp Gly Arg Ala Pro Ser Thr Ala Val Ser Thr Gly
        35                  40                  45

Phe Leu Thr Tyr Ala Ala His Glu Gly Phe Asn Arg Ala Gln Leu Tyr
    50                  55                  60

Glu Ala Met Ser Gln Thr Thr Gly Lys Gly Leu Cys Asp Val Ala Leu
65                  70                  75                  80

Leu Arg Arg Leu Val Asp Glu Ala Pro Lys Glu Met Ala Glu Leu Ile
                85                  90                  95

Glu Thr Tyr Lys Val Pro Val Asp Asn Thr Glu Arg Gly Val Arg Ala
            100                 105                 110

Arg Arg Ala Val Gly Lys Ser Gly Lys Glu Leu Leu Ser Gly Leu Asp
        115                 120                 125

Ala Asp Tyr Gly Thr Arg Gly Ser Leu Glu Asp Thr Thr Gly Leu Met
    130                 135                 140

Met Glu Phe Ser Ser Thr His Gly Thr Ala Leu Tyr Ala Gln Leu Arg
145                 150                 155                 160

Lys Ala Val Asn Thr Ala Pro Lys Ile Arg Arg Val Arg Gly Ser Ala
                165                 170                 175

Leu Val Leu Glu Pro Gly Ser Thr Thr Val Gly Ala Leu Val Asp Gly
            180                 185                 190

Glu Pro Val Thr Ile Ala Ala Arg Ser Ile Ile Leu Ala Thr Gly Gly
        195                 200                 205

Ile Gln Gly Leu Tyr Glu Val Thr Asp Asn Pro His Thr Leu Thr Gly
    210                 215                 220

Asp Gly His Gly Met Ala Met Asp Ala Gly Ala Glu Phe Val Asp Met
225                 230                 235                 240

Glu Phe Met Gln Phe Tyr Pro Leu Ser Val Asn Glu Glu Gly Ala Pro
                245                 250                 255

Thr Leu Phe Phe Tyr Pro Asp Phe Pro Arg Arg Ala Lys Leu Ile Asp
            260                 265                 270

Asp Gly Gly Arg Asn Val Leu Val Lys His Leu Gly Glu Gly Ser Gln
        275                 280                 285

Tyr Leu Ser Glu Leu His Asn Trp Asp Gln Leu Ala Ala Val Val Gln
    290                 295                 300

Thr Glu Ile Val Glu Gly Arg Lys Val Phe Val Asp Phe Arg Glu Thr
305                 310                 315                 320

Lys Pro Glu Glu Trp Ala Pro Asp Ser Leu Thr Gly Thr Phe Leu Gly
                325                 330                 335

Lys Cys Val Pro Asn Phe Met Thr Thr Pro Val Gln Val Ala Pro Ser
            340                 345                 350

Ser His Tyr Thr Ile Gly Gly Leu Lys Val Asp Val Asp Gly Arg Thr
        355                 360                 365

Asn Leu Pro Lys Leu Tyr Ala Val Gly Glu Leu Ala Gly Gly Val His
    370                 375                 380

Gly Ala Asn Arg His Gly Gly Thr Ala Leu Val Asp Ala Met Thr Tyr
385                 390                 395                 400

Gly Arg Ile Ala Gly Arg His Ala Ala Gly Ser Leu Asn Gly Ala Ala
                405                 410                 415

Ala Thr Gly Gly Ala Ala Leu Leu Pro Ala Gly Ser Lys Ala Gly Lys
            420                 425                 430

Ala Ser Arg Ile Glu Gly Ala Met Ser Asp Leu Arg Arg Ala Asn Gln
        435                 440                 445

Leu Ala Leu Gly Pro Ile Arg Asp Ala Val Arg Leu Gln Arg Val Gly
    450                 455                 460

Glu Leu Phe Ala Glu Leu Leu Asp Glu Val Arg Ser Phe Gly Trp Asn
465                 470                 475                 480

Gly Tyr Lys Glu Met Gln Glu Ile Leu Arg Val Glu Arg Ala Ile Lys

485                    490                    495

Leu Ser Asp Ala Met Arg Gln Ala Met Leu Arg Arg Thr Glu Thr Arg
            500                    505                    510

Gly Val His Tyr Arg Ala Asp Phe Pro Ser Ser Ser Asp Ala Trp Leu
        515                    520                    525

Lys Lys Gln Val Phe Ala Leu Arg Asp Gly Ala Leu His Phe Lys Asp
    530                    535                    540

Val Pro Leu
545

<210>  22
<211>  24
<212>  DNA
<213>  Artificial

<220>
<223>  Forword primer for Asymmetric reductase coding region

<400>  22
caccatgtcg gatgttcttg taac                                              24

<210>  23
<211>  19
<212>  DNA
<213>  Artificial

<220>
<223>  Reverse primer for Asymmetric reductase coding region

<400>  23
ctacgcttgc ggaagcaaa                                                    19

## Description

### TECHNICAL FIELD

The present invention relates to a reductase gene for an α-substituted-α,β-unsaturated carbonyl compound, and enzymes as gene products thereof. More specifically, the present invention relates to a reductase gene for an α-substituted-α,β-unsaturated carbonyl compound having activities of producing a corresponding α-substituted-α,β-saturated carbonyl compound by hydrogenating an α, β-carbon-carbon double bond of an α-substituted carbonyl compound characterized in that the gene is derived from at least one microorganism selected from the group consisting of the genus Acetobacter, Actinomyces, Acinetobacter, Agrobacterium, Aeromonas, Alcaligenes, Arthrobacter, Azotobacter, Bacillus, Brevibacterium, Burkholderia, Cellulomonas, Corynebacterium, Enterobacter, Enterococcus, Escherichia, Flavobacterium, Gluconobacter, Halobacterium, Halococcus, Klebsiella, Lactobacillus, Microbacterium, Micrococcus, Micropolyspora, Mycobacterium, Nocardia, Pseudomonas, Pseudonocardia, Rhodococcus, Rhodobacter, Serratia, Staphylococcus, Streptococcus, Streptomyces, and Xanthomonas, and enzymes as gene products thereof.

Further, the present invention relates to a reductase gene for an α-substituted-α, β-unsaturated carbonyl compound derived from genus Pseudomonas or Burkholderia microorganisms, in particular Pseudomonas sp. SD810 strain, Pseudomonas sp. SD811 strain, Pseudomonas sp. SD812 strain, and Burkholderia sp. SD816 strain, or enzymes as gene products thereof having the above activities.

Furthermore, the present invention relates to a reductase gene and an enzyme as a gene product thereof, which are useful in producing an α-substituted-α,β-saturated carbonyl compound optically active at the α-position by stereoselectively hydrogenating a carbon-carbon double bond in a corresponding α-substituted carbonyl compound having an α, β-carbon-carbon doublebond, which is a molecule prochiral at the α-position.

The novel enzyme gene and the enzyme as the product thereof can be used in the field of production of optically active carbonyl compounds including various optically active saturated carboxylic acids (having the S- or R-form absolute configurations at their respective α-positions with substituted groups, respectively) or amides. The optically active carbonyl compounds are highly valuable chiral building blocks, which can be hardly prepared by classical chemical processes, and in particular the compounds are useful materials as raw materials of medical and agricultural chemicals.

### BACKGROUND ART

In recent years, much attention has been paid on the method of producing various compounds, particularly optically active substances, by the microbial reduction of carbon-carbon double bonds. To this end, many publications have reported various processes of producing a corresponding α,β-saturated carbonyl compound having a substituent at the α-position from a carbonyl compound having an α,β-carbon-carbon double bond and having a substituent at the α-position by microbially reducing the carbon-carbon double bond (See e.g., Hoppe-Seyler's Z. Physiol. Chem. 362, 33 (1981); Arch. Microbiol. 135, 51 (1983); Helv. Chim, Acta., 62, 455 (1979); J. Ferm. Bioeng., 84, 195 (1997)).

However, no example has been provided with respect to the separation and identification of reductase from active microorganisms used in these processes. Firstly, few studies have been performed on enzymes belonging to this group because of difficulties in separation and identification due to their instability. The enzyme of the present invention has not been an exceptional case, so that the separation and identification of the enzyme has been impossible in the conventional process because of its rapid inactivation.

On this account, such a disadvantage has been difficult to mitigate by a genetic- or metabolic-engineering approach when the reductase is used in the production of a chemical compound. Therefore, an effective improvement in production process has been hardly conducted.

### DISCLOSURE OF THE INVENTION

Therefore, an object of the present invention is to provide a catalytic enzyme useful in producing a corresponding α-substituted-α,β-saturated carbonyl compound from an α-substituted carbonyl compound having an α, β-carbon-carbon double bond by microbial reduction of the carbon-carbon double bond and to provide a gene of the catalytic enzyme.

Thorough screening from soil has allowed the inventors of the present invention to find that, surprisingly, microorganisms, each of which is capable of producing a corresponding α-substituted-α,β-saturated carbonyl compound from an α-substituted carbonyl compound having an α, β-carbon-carbon double bond by reduction of the carbon-carbon double bond, are distributed over a relatively wide genus range of the aerobic and facultative anaerobic bacteria (e.g., JP 10-224821 A).

In particular, it has been found that a large number of strains having the above enzymatic activity are present in microorganisms belonging to the genera Pseudomonas and Burkholderia, and some of these strains can reduce an α-halocarbonyl compound having an α,β-carbon-carbon double bond to thereby produce an extremely high-purity α-halo-α,β-saturated carbonyl compound having the S absolute configuration at the α-position.

Furthermore, the inventors of the present invention have succeeded in establishing a method of producing optically active α-substituted-α,β-saturated carbonyl compounds using these active microorganisms and dedicated to studying for identification of reductase itself and also for identification of a gene thereof to improve the production process. As a result, the inventors of the present invention have succeeded in identifying a catalytic enzyme, revealing the action mechanism of the reductase, and collecting microorganisms having high activity, resulting in the completion of the present invention.

In other words, the present invention relates to a reductase gene, plasmid, transformant, a protein, a method of producing a gene that encodes the protein, and a reductase gene for an α, β-unsaturated carbonyl compound.
1. A gene including: DNA having a base sequence represented by SEQ ID NO: 19 that encodes a protein having a reduction activity to an α-substituted-α,β-unsaturated carbonyl compound; or DNA that hybridizes with the DNA under stringent conditions.
2. A gene including: DNA having a base sequence represented by SEQ ID NO: 17 that encodes a protein having a reduction activity to an α-substituted-α,β-unsaturated carbonyl compound; or DNA that hybridizes with the DNA under stringent conditions.
3. A gene including: DNA having a base sequence represented by SEQ ID NO: 18 that encodes a protein having a reduction activity to an α-substituted-α,β-unsaturated carbonyl compound; or DNA that hybridizes with the DNA under stringent conditions.
4. A reductase gene for an α-substituted-α,β-unsaturated carbonyl compound, characterized by including a DNA sequence encoding an amino acid sequence represented by SEQ ID NO: 20 and an amino acid sequence represented by SEQ ID NO: 21.
5. A gene that encodes a protein having a reduction activity to an α-substituted-α,β-unsaturated carbonyl compound, comprising an amino acid sequence represented by SEQ ID NO: 20 or an amino acid sequence having deletion, substitution, or addition of one or more amino acids.
6. A gene that encodes a protein having a reduction activity to an α-substituted-α,β-unsaturated carbonyl compound, comprising an amino acid sequence represented by SEQ ID NO: 21 or an amino acid sequence having deletion, substitution, or addition of one or more amino acids.
7. A reductase gene for an α-substituted-α, β-unsaturated carbonyl compound according to any one of 1 to 6, in which the reductase gene for an α-substituted-α,β-unsaturated carbonyl compound is derived from at least one microorganism selected from the group consisting of the genus Acetobacter, Actinomyces, Acinetobacter, Agrobacterium, Aeromonas, Alcaligenes, Arthrobacter, Azotobacter, Bacillus, Brevibacterium, Burkholderia, Cellulomonas, Corynebacterium, Enterobacter, Enterococcus, Escherichia, Flavobacterium, Gluconobacter, Halobacterium, Halococcus, Klebsiella, Lactobacillus, Microbacterium, Micrococcus, Micropolyspora, Mycobacterium, Nocardia, Pseudomonas, Pseudonocardia, Rhodococcus, Rhodobacter, Serratia, Staphylococcus, Streptococcus, Streptomyces, and Xanthomonas.
8. A reductase gene for an α-substituted-α,β-unsaturated carbonyl compound according to 7, in which the reductase gene for an α-substituted-α,β-unsaturated carbonyl compound is derived from a Pseudomonas microorganism.
9. A reductase gene for an α-substituted-α, β-unsaturated carbonyl compound according to 7, in which the reductase gene for an α-substituted-α,β-unsaturated carbonyl compound is originated from a Burkholderia microorganism.
10. A reductase gene for an α-substituted-α, β-unsaturated carbonyl compound according to 8, in which the Pseudomonas microorganism is Pseudomonas sp. SD810 strain.
11. A reductase gene for an α-substituted-α, β-unsaturated carbonyl compound according to 8, in which the Pseudomonas microorganism is Pseudomonas sp. SD811 strain.
12. A reductase gene for an α-substituted-α, β-unsaturated carbonyl compound according to 8, in which the Pseudomonas microorganism is Pseudomonas sp. SD812 strain.
13. A reductase gene for an α-substituted-α, β-unsaturated carbonyl compound according to 9, in which the Burkholderia microorganism is Burkholderia sp. SD816 strain.
14. A reductase gene for an α-substituted-α, β-unsaturated carbonyl compound according to any one of 1 to 13, in which the reductase has a catalytic activity to reduce a carbon-carbon double bond to produce an S-form compound chiral at an α-position.
15. A reductase gene for an α-substituted-α, β-unsaturated carbonyl compound according to any one of 1 to 14, in which:
   the α-substituted-α,β-unsaturated carbonyl compound is a compound represented by the following general formula (1) 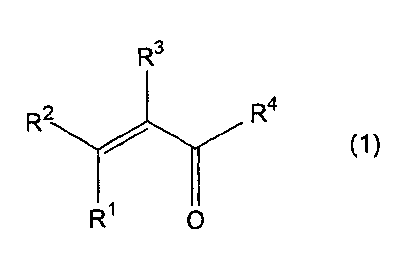 wherein R¹, R², and R³ each independently represent a hydrogen atom, a halogen atom, a linear or branched aliphatic hydrocarbon group having 1 to 6 carbon atoms, a linear or branched alkoxy group having 1 to 6 carbon atoms, a hydroxyl group, a carboxyl group, an aromatic group or a nitrogen-, oxygen-, or sulfur-containing heterocyclic group which may be substituted, and R⁴ represents a hydroxyl group, a linear or branched alkoxy group having 1 to 3 carbon atoms, or a primary, secondary, or tertiary amino group, provided that R³ is not a hydrogen atom; and
   a reduced compound is a compound represented by the following general formula (2) 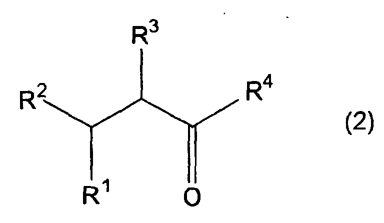 wherein R¹ to R⁴ have the same meanings as those defined above.
16. A reductase gene for an α-substituted-α, β-unsaturated carbonyl compound according to 15, in which:
   the α-substituted-α,β-unsaturated carbonyl compound is an α-haloacrylic acid represented by the following general formula (1) 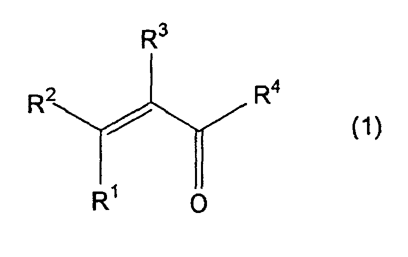 wherein R¹ and R² represent hydrogen atoms, R³ represents a halogen atom, and R⁴ represents a hydroxyl group; and
   the reduced compound is an α-halopropionic acid having an S absolute configuration represented by the following general formula (2) 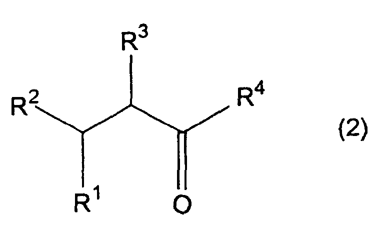 wherein R¹ to R⁴ have the same meanings as those defined above.
17. A reductase gene for an α-substituted-α, β-unsaturated carbonyl compound according to 16, in which R³ represents a bromine atom.
18. A reductase gene for an α-substituted-α, β-unsaturated carbonyl compound according to 16, in which R³ represents a chlorine atom.
19. A reductase gene for an α-substituted-α, β-unsaturated carbonyl compound according to 16, in which R³ represents a fluorine atom.
20. A plasmid, characterized by containing a DNA of a reductase gene for an α-substituted-α,β-unsaturated carbonyl compound according to any one of 1 to 19.
21. A plasmid, characterized by containing a reductase gene for an α-substituted-α,β-unsaturated carbonyl compound according to any one of 1 to 19 and a gene for an enzyme functioning with an NADPH as a co-enzyme.
22. A transformant transformed with a plasmid according to 20 or 21.
23. A transformant including a product transformed by a plasmid according to 20, and a plasmid containing a gene for an enzyme functioning with an NADPH as a co-enzyme.
24. A protein having an activity to reduce an α-substituted-α, β-unsaturated carbonyl compound wherein the proten is an expression product of a reductase gene for the α-substituted-α,β-unsaturated carbonyl compound according to any one of 1 to 19, or a protein having deletion, substitution, or addition of one or more amino acids thereof and having a reduction activity to an α-substituted-α,β-unsaturated carbonyl compound.
25. A protein having an activity to reduce an α-substituted-α,β-unsaturated carbonyl compound containing an amino acid sequence represented by SEQ ID NO: 20 or an amino acid sequence having deletion, substitution, or addition of one or more amino acids in the said amino acid sequence.
26. A protein having an activity to reduce an α-substituted-α,β-unsaturated carbonyl compound containing an amino acid sequence represented by SEQ ID NO: 21 or an amino acid sequence having deletion, substitution, or addition of one or more amino acids in the said amino acid sequence.
27. A method of producing a gene that encodes a protein having an activity to reduce an α-substituted-α,β-unsaturated carbonyl compound, comprising using a pair of primers prepared by combining a base sequence selected from base sequences located upstream of abase at position 631 and abase sequence selected frombase sequences located downstream of a base at position 3, 543 in the base sequence represented by SEQ ID NO: 19, where both the base sequences extend in opposite directions to each other.
28. A method of producing a gene that encodes a protein having an activity to reduce an α-substituted-α,β-unsaturated carbonyl compound, comprising using a pair of primers prepared by combining a base sequence selected from base sequences located upstream of a base at position 631 and a base sequence selected from base sequences located downstream of a base at position 2,274 in base sequences represented by SEQ ID NO: 19, where both base sequences extend in opposite directions to each other.
29. A method of producing a gene that encodes a protein having an activity to reduce an α-substituted-α,β-unsaturated carbonyl compound, comprising using a pair of primers prepared by combining a base sequence selected from base sequences located upstream of a base at position 2,547 and a base sequence selected from base sequences located downstream of a base at position 3,543 in base sequences represented by SEQ ID NO: 19, where both base sequences extend in opposite directions so as to be reversed strands with respect to each other.
30. Amethod of reducing an α-substituted-α,β-unsaturated carbonyl compound, comprising:
   using a culture and/or treated product of a transformant according to 22 or 23.

### BRIEF DESCRIPTION OF THE DRAWINGS

Fig. 1 is a graph showing different reaction curves with different carbon sources in the cultures of Pseudomonas sp. SD811 strain;
Fig. 2 is a graph showing different reaction curves with different carbon sources in the cultures of Burkholderia sp. SD816 strain;
Fig. 3 is a two-dimensional electrophoretic image that shows the results in the comparison among proteins from cultures of Burkholderia sp. SD816 strain with different carbon sources;
Fig. 4 is a schematic diagram that represents a positional relationship between a CAA43 gene and a CAA67 gene and also represents the positions of the respective DNA fragments (the number of each fragment represents SEQ ID NO); and

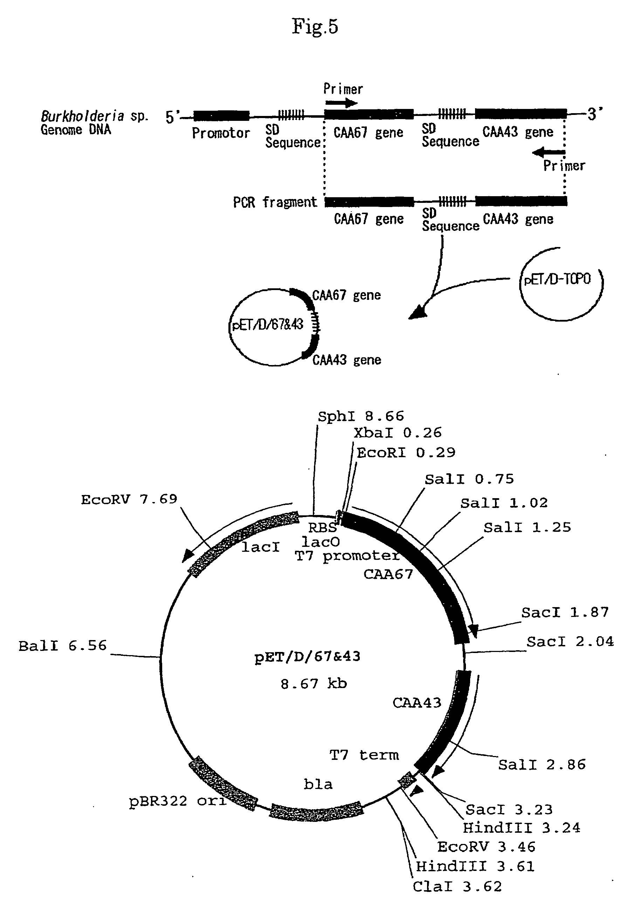

Fig. 5 is a schematic diagram that represents the construction of an expression vector for a reductase gene responsible for asymmetric reduction.

### DETAILED DESCRIPTION OF THE INVENTION

A reductase gene for an α-substituted- α,β-unsaturated carbonyl compound and enzymes as products there of according to the present invention exist in microorganisms belonging to any one of the genus Acetobacter, Actinomyces, Acinetobacter, Agrobacterium, Aeromonas, Alcaligenes, Arthrobacter, Azotobacter, Bacillus, Brevibacterium, Burkholderia, Cellulomonas, Corynebacterium, Enterobacter, Enterococcus, Escherichia, Flavobacterium, Gluconobacter, Halobacterium, Halococcus, Klebsiella, Lactobacillus, Microbacterium, Micrococcus, Micropolyspora, Mycobacterium, Nocardia, Pseudomonas, Pseudonocardia, Rhodococcus, Rhodobacter, Serratia, Staphylococcus, Streptococcus, Streptomyces, and Xanthomonas.

Preferably they are derived from Pseudomonas or Burkholderia microorganisms.

Original microorganisms used in the present invention may be any strains as far as they have the activity of reducing an α,β-carbon-carbon double bond of an α-substituted carbonyl compound having an α,β-carbon-carbon double bond. Preferable examples of the microorganisms include, but not particularly limited to, Pseudomonas sp. SD810 strain, Pseudomonas sp. SD811 strain, Pseudomonas sp. SD812 strain, and Burkholderia sp. SD816 strain.

Among those, Pseudomonas sp. SD811 strain or Burkholderia sp. SD816 strain is particularly preferably used in terms of comparatively high reduction activity.

The microorganisms used, such as Pseudomonas sp. SD810 strain, Pseudomonas sp. SD811 strain, Pseudomonas sp. SD812 strain, and Burkholderia sp. SD816 strain, which are isolated from soil, have their own activities of decomposing and assimilating various carbonyl compounds.

These microorganisms, Pseudomonas sp. SD810 strain, Pseudomonas sp. SD811 strain, and Pseudomonas sp. SD812 strain, were deposited with the National Institute of Bioscience and Human-Technology under the accession numbers BP-6767 (FERMBP-6767) (transferred from accession number 16746 (FERM-16746)), BP-6768 (FERM BP-6768) (transferred from accession number 16747 (FERM-16747)), and BP-6769 (FERM BP-6769) (transferred from accession number 16748 (FERM-BP-6769)), respectively. In addition, Burkholderia sp. SD816 strain is deposited with the National Institute of Bioscience and Human-Technology under the accession number BP-6770 (FERM BP-6770).

Those strains may be isolated and cultured by the conventional procedures including those specifically described in JP 10-224821 A.

The active microorganisms described above may show variations in their reduction activities depending on their culture conditions. That is, each of the microorganisms shows different activities on the reduction of an α,β-carbon-carbon double bond between the case where the microorganism is cultured using an α-substituted carbonyl compound having an α,β-carbon-carbon double bond (i.e., reduction substrate) as a carbon source and the case where the microorganism is cultured using a typical carbon source such as a saccharide. That is, a microorganism cultured using a reduction substrate as a carbon source may show a high reduction activity from the beginning of the reaction. It suggests that the reductase is induced partly or wholy with the reduction substrate, so that an analysis on such a difference will lead to the identification of the reductase.

A carbon source in a culture medium for obtaining a microorganism having a high reduction activity may be a compound represented by the general formula (1).

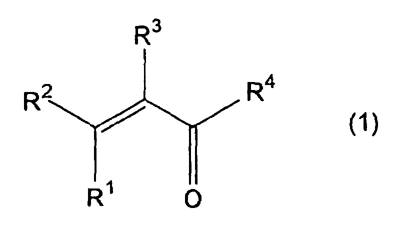

In the formula (1) , R¹ and R² each independently represent a hydrogen atom, a halogen atom, a linear or branched aliphatic hydrocarbon carbon group having 1 to 6 carbon atoms, a linear or branched alkoxy group having 1 to 6 carbon atoms, a hydroxyl group, a carboxyl group, an aromatic group or a saturated or unsaturated nitrogen-, oxygen-, or sulfur-containing heterocyclic group which may be substituted. Preferably R¹ and R² are hydrogen atoms;

R³ represents a halogen atom, a linear or branched aliphatic hydrocarbon group having 1 to 6 carbon atoms, a linear or branched alkoxy group having 1 to 6 carbon atoms, a hydroxyl group, a carboxyl group, an aromatic group or a saturated or unsaturated nitrogen-, oxygen-, or sulfur-containing heterocyclic group which may be substituted, preferably a halogen atom, in particular a chlorine atom or a bromine atom.

R⁴ represents a hydroxyl group, a linear or branched alkoxy group having 1 to 4 carbon atoms, or a primary, secondary, or tertiary amino group, preferably a hydroxyl group.

Specific examples of the compound include α-chloroacrylic acid, α-bromoacrylic acid, 2-chloro-2-butenoic acid, 2-bromo-2-butenoic acid, 2-chloro-2-pentenoic acid, 2-bromo-2-pentenoic acid, and methyl esters and ethyl esters thereof. Of these, α-chloroacrylic acid and α-bromoacrylic acid are preferred.

More specifically, bacterial cells having a high reduction activity can be obtained by: inoculating a strain in 5 ml of minimal medium prepared by adding 2 g/l of an α,β-unsaturated carbonyl compound having a substituent at the α-position, such as α-chloroacrylic acid, as a substantially only carbon source to an inorganic salt culture medium (e.g., (NH₄)₂SO₄: 2 g/l, NaH₂PO₄: 1 g/l, K₂HPO₄: 1 g/l, MgSO₄: 0.1 g/l, yeast extract: 0.5 g/l) used for normal bacteria; and incubating the bacteria at 28°C for 12 to 72 hours while shaking. On the other hand, when the bacteria cells are incubated such that only the carbon source in the above culture conditions is replaced with a metabolic product of the reduction substrate, for example lactic acid when the carbon source is a substituted acrylic acid such as α-chloroacrylic acid, bacterial cells having no reduction activity can be obtained at the beginning of the reaction.

These bacterial cells are collected by centrifugation and disrupted by the conventional method such as French press to obtain a cell-free extract. Then, the cell-free extract is subjected to column chromatography to make a comparison between the migration patterns of separated proteins, exhibiting different proteins between the bacterial cells incubated under different conditions.

Among the proteins produced from the bacterial cells incubated using the reduction substrate, proteins having increased amounts of production may be isolated and then the activity thereof may be measured, allowing the identification of the desired enzymes. In general, however, such enzymes show low stability in the state of a cell-free extract. Therefore, the activities of the enzymes disappear comparatively quickly, so that the separation and identification of the enzymes will be difficult in many cases. This fact is one of the causes involved in stagnation in research on enzymes belonging to the group of the above enzymes compared with other stable enzymes.

In this case, the activity of the enzyme may be retained by carrying out the isolation procedures under nitrogen atmosphere. Alternatively, however, there is an effective process in which a partial sequence of a gene is revealed, a target gene is cloned using a DNA base sequence estimated from the partial sequence as a probe, and the gene is then expressed to obtain a significant amount of the protein, followed by analyzing the protein for its activity or the like.

In other words, the production patterns of proteins separated from cell-free extracts using different carbon sources by two-dimensional protein electrophoresis or the like are compared and then a protein being increased in bacterial cells incubated with a reduction substrate is found. Subsequently, the protein thus obtained is transferred to a PVDF membrane or the like, followed by analyzing the N-terminal sequence of the protein using a vapor-phase Edman degradation apparatus or the like. A DNA base sequence is estimated from the resulting N-terminal sequence and the corresponding oligonucleotide is then synthesized to prepare a probe useful for acquiring genes for a group of reductase enzymes from chromosomes (i.e., a DNA fragment labeled with an identifiable marker, which can be used for finding out DNA having a specific sequence).

The reductase gene of the present invention can be easily obtained by the conventional methods such as Southern hybridization generally used in genetic engineering using a DNA probe prepared as described above. More specifically, DNA extracted from the above microorganism (including plasmid if the DNA exists in chromosome and in plasmid) is cut into fragments by appropriate restriction enzymes. The resulting fragments are separated in size by means of agarose gel electrophoresis or the like and then transferred on a nitrocellulose membrane, followedby subjecting the transferred fragments to hybridization with a probe labeled with an identifiable marker (here, the term "hybridization" means the formation of a double strand DNA when there is high base complementarity between DNA sequences, and is also referred to as "pairing"), resulting in a fragment that hybridizes the probe in a specific manner, or a DNA fragment that contains a target gene. In this case, although the gene may be cut into partial fragments, the entire gene can be obtained by employing the same detection method with different kinds of restriction enzymes, using a previously obtained fragment as a probe, or the like.

If a hybridization method is applied on genes for a group of reductases of the present invention, although appropriate conditions may be different depending on the length of DNA to be hybridized, a sufficiently specific hybridization result will be obtained under stringent conditions of about 40°C to 70°C, preferably 47°C to 60°C within a salt concentration range of a typical hybridization solution.

The genes for a group of reductases of the present invention can be also obtained easily by forming primers that hybridize on appropriate sites of the genes and peripheral sequences of the genes; and performing a polymerization chain reaction (PCR) using the microbial DNA as a template.

The term "primer" used herein is a fragment that is hybridized on a target DNA sequence to be replicated and functions as the initiation point of DNA synthesis. A primer is indispensable in initiation of DNA replication because enzymatic DNA synthesis proceeds such that DNA polymerase catalyses the diester-binding of deoxyribonucleotide on the 3'-OH position of the primer hybridized on the template DNA. A primer is used even for a polymerase chain reaction (PCR) , where efficient replication of the target DNAdepends on the selection of such a primer.

A primer, which can be used in the present invention, is not limited to specific one as far as it will be hybridized on the reductase gene of the present invention and the peripheral sequence of the gene and will function as the initiation point for DNA synthesis. For example, there are no limitations on the degree of the sequence complementarity of the fragment, the length of the fragment, modifications to the fragment, and the like. For any purpose, for example, a primer that contains an adaptor sequence for connecting a fragment generated to a plasmid, a primer modified by a fluorescent substance for facilitating the detection of a gene fragment generated, or the like can be designed and used at will.

A pair of primers useful for obtaining genes for a group of reductases in the present invention is a combination of one having a base sequence containing a sequence upstream of the base at position 631, which is a first base of the initiation codon of the upstream gene among the base sequences represented in SEQ ID NO: 19, and the other having a base sequence downstream of the base at position 3, 543, which is a thirdbase of the termination codon of the downstream gene, such that the primer strands extend in opposite directions to each other. Another pair of primers useful for obtaining genes for a group of reductases is a combination of one having a base sequence containing a sequence upstream of the base at position 631, which is a first base of the initiation codon of the upstream gene among the base sequences represented in SEQ ID NO: 19, and the other having a base sequence downstream of the base at position 2,274, which is a third base of the termination codon of the upstream gene, such that the primer strands extend in opposite directions to each other. Further another pair of primers useful for obtaining genes for a group of reductases is a combination of one having a base sequence containing a sequence upstream of the base at position 2, 542, which is a first base of the initiation codon of the downstream gene among the base sequences represented in SEQ ID NO: 19, and the other having a base sequence downstream of the base at position 3, 543, which is a thirdbase of the termination codon of the downstream gene, such that the primer strands extend in opposite directions to each other. Those three combinations provide DNA fragments each containing one of the entire gene group, upstream gene, and downstream gene. Furthermore, there is also a useful combination of primers, which are prepared such that base sequences having over ten or several tens of bases are provided on both ends of the base sequence represented by SEQ ID NO: 17, which extend in opposite directions to each other. This combination allows the production of DNA that corresponds to the base sequence represented by SEQ ID NO: 17, so that a gene corresponding to the downstream gene of the present invention can be produced. Similarly, there is also a useful combination of primers, which are prepared such that base sequences having over ten or several tens of bases are provided on both ends of the base sequence represented by SEQ ID NO: 18, extending in opposite directions to each other. This combination allows the production of DNA that corresponds to the base sequence represented by SEQ ID NO: 18, so that a gene corresponding to the upstream gene of the present invention can be produced.

A procedure for obtaining genes using those primers is not specificallylimited. However, the polymerase chain reaction (PCR) can be most convenient. The reaction conditions are not specifically limited as far as the DNA synthetic reaction produces a reaction product. Conventionally, the reactionmaybe performed by combining appropriate conditions of a denature temperature of generally 90°C to 100°C, preferably 94°C to 98°C, an annealing temperature of 30°C to 70°C, preferably 37°C to 65°C, more preferably 5°C higher than Tm of the primer, and an extension temperature of 65°C to 75°C, preferably 72°C. The number of reaction cycles may be usually selected from about 15 to 50 cycles even though the reaction can be repeated until the desired amount of the product will be obtained. The sequence of the gene obtained may be one of the closely-related variants having their own portions different from each other as a result of the sequence of the DNA strand used as a template and the strength of proof-reading function of DNA polymerase used in the synthesis (the mechanism by which a base incorporated by mistake at the time of DNA replication is removed by the 5' to 3' exonuclease activity of DNA polymerase). However, the closely-related reductase genes can be used in the present invention just as in the case of the original reductase gene used as an origin for primer designing.

These genes are introduced into the host organisms such that the genes can be expressed in the bodies of the host organisms using expression vectors generally known in the art, allowing the production of organisms each having a high reduction activity enough to produce a corresponding α-substituted-α,β-saturated carbonyl compound from an α-substituted carbonyl compound having an α,β-carbon-carbon double bond by reducing the carbon-carbon double bond. At this time, the downstream gene can obtain a reduction activity when the downstream gene is not used by itself but is combined with the upstream gene.

Examples of microorganisms for expressing the reductase gene of the present invention are not particularly limited and examples thereof include microorganisms in which host vectors are developed such as bacteria including Escherichia, Bacillus, Pseudomonas, Serratia, Brevibacterium, Corynebacterium, Streptococcus, and Lactobacillus; yeasts such as Saccharomyces, Kluyveromyces, Schizosaccharmyces, Zygosasccharomyces, Yarrowia, Trichosporon, Rhodosporidium, Hansenula, Pichia, and Candida; and fungi such as Neurospora, Aspergillus, Cephalosporium, and Trichoderma. One example of the preferable microorganisms is Escherichia coli. The active microorganism produced does not require any culture medium that contains the above enzyme-inducing substrate as a carbon source. The active microbial cells can be obtained by culturing the cells in a general nutrient culture medium such as an LB medium.

The reduction reaction using the reduction-active microorganism produced can be performed under the conditions just as in the case of the reaction of the microorganism, from which the present enzyme is derived, disclosed in JP 2000-106891 A.

In other words, as far as the reducing power of the microorganism can be stably expressed, the reaction for reducing an α,β carbon-carbon double bond of an α-substituted carbonyl compound having the-carbon-carbon double bond may be performed in a culture medium of the microorganism, or performed using cells obtained by the above process, the product obtained by processing the microorganism such as a cell-free extract obtained by disrupting microbial cells cultured by the above process, or the like.

More specifically, in the case of using the cultured microbial cells, an α-substituted carbonyl compound having an α,β-carbon-carbon double bond to act as a substrate is added continuously or batchwise to a culture medium in a concentration of 0.1 to 10% by mass, preferably 0.2 to 2% by mass, and is then incubated at a culture temperature of 15 to 40°C, preferably 25 to 37°C, thereby producing a corresponding α-substituted-α, β-saturated carbonyl compound in the culture medium.

Alternatively, the culture obtained by the above method is subjected to centrifugation or the like to collect microbial cells, and the cells are then suspended in an appropriate solution, for example, an aqueous solution such as a diluted pH buffer. Then, the suspension is added with an α-substituted carbonyl compound having an α,β-carbon-carbon double bond as a substrate continuously or batchwise in a concentration of, for example, 0.1 to 10% by mass, preferably 0.2 to 2% by mass at a reaction temperature of 15 to 50°C, preferably from 25 to 37°C, more preferably 28 to 35°C, while adjusting the reaction pH to 6.0 to 9.0, preferably from 6.5 to 7.3, thereby producing a corresponding α-substituted-α, β-saturated carbonyl compound in the microbial cell suspension. The pH is preferably maintained constant by means of an aqueous buffer such as one containing potassium phosphate or tris/HCl in a concentration of 10 mM to 1 M.

The timing and rate or frequency of the addition of the α-substituted carbonyl compound having an α, β-carbon-carbon double bond may be freely selected as far as the reaction can be completed within the target time.

In the case of using a processed microbial product, for example, the culture obtained by the above culture method is subjected to centrifugation to collect microbial cells, and then the cells are disrupted by French pressing or the like to obtain a cell-free extract. Then, the cell-free extract is added to a reaction solution containing an α-substituted carbonyl compound having an α,β-carbon-carbon double bond as a substrate in a concentration of 0.1 to 10% by mass, preferably from 0.2 to 2% by mass, and also containing 10 mM to 1 M of an ingredient effective in maintaining the pH of the reaction solution. Subsequently, a reaction is carried out at a temperature of 15 to 50°C, preferably 28 to 35°C, therebyproducing a corresponding α-substituted-α,β-saturated carbonyl compound.

In the present invention, the reaction may be performed while a substance (e.g., a compound capable of being oxidized by the microorganism used, such as saccharide or organic acid, preferably glucose or L-lactic acid), which is effective in maintaining the activity of reducing an α-substituted carbonyl compound having an α,β-carbon-carbon double bond by itself or a mixed solution with an α-chloroacrylic acid is added continuously or batchwise such that the concentration of the substance reaches 0.1 to 10% by mass, preferably 0.2 to 1% by mass during the reaction. The ratio of the α-chloroacrylic acid to the added substance to be oxidized may be freely selected between 1:1 and 20:1 on a molar basis. The addition of saccharide or organic acid prolongs a reaction time, allows an increase in the concentration of the target product, an α-halo-α,β-saturated carbonyl compound, in the reaction solution and is advantageous for collecting the product by isolation. In particular, for improving a system for efficiently reproducing the reduction type of co-enzyme NADPH using the substance to be oxidized, an appropriate oxidation-reduction enzyme gene, such as a malate dehydrogenase gene or a glutamate dehydrogenase gene, may be introduced into a microorganism so as to be expressed together with the reductase gene to significantly improve productivity. Such a method is disclosed in publications such as JP 61-128895 A and Biotechnol. Genet. Eng. Rev. 6, 221-270 (1988).

The reactionmaybe carried out either in an aerobic or anaerobic environment when the bacterial cells are not in culture. The ratio of the bacterial cells or cell-free extract to the α-substituted halocarbonyl compound having an α,β-carbon-carbon double bond as the substrate, or the timing and rate or frequency of addition of the substrate may be freely selected as far as the reaction can be completed within the desired time.

In the present invention, an α-substituted-α,β-saturated carbonyl compound produced by the reduction of an α-substituted carbonyl compound having an α,β-carbon-carbon double bond is a metabolic intermediate for the microorganism used and may be further decomposed. In such a case, the decomposition reaction may be terminated by selecting or preparing a host microorganism having no decomposition activity.

Furthermore, cells or cell-free extract of the microorganism for use in the present invention may be used by fixing the cells or extract to an immobilizing support of various types by a commonly known method such as adsorption, inclusion, or cross-linking. The supports to be used include, but not specifically limited to, polysaccharide-based materials such as cellulose, polymer-based materials, and protein-based materials such collagen.

The α-substituted-α,β-saturated carbonyl compound produced according to the present invention may be isolated and purified using an ordinary purification method such as solvent extraction or distillation. For example, α-chloropropionic acid produced from α-chloroacrylic acid may be obtained by subjecting the culture or reaction solution to organic solvent extraction, distillation, or the like. Furthermore, although an α-substituted carbonyl compound having an α,β-carbon-carbon double bond is a molecule prochiral at the α-position, the purity of an enantiomer of the α-substituted-α,β-saturated carbonyl compound produced by the reducing method of the present invention, which is a chiral compound, can be determined by means of GC or HPLC with a chiral column or by means of a polarimeter.

As described above, the present invention provides a group of reductases useful for producing a corresponding α-substituted-α, β-saturated carbonyl compound having an S absolute configuration from an α-substituted carbonyl compound having an α,β-carbon-carbon double bond by reducing the carbon-carbon double bond and also provides a group of genes of the reductases. Furthermore, the present invention provides a manufacturing process using a high-productive organism obtained by the use of those genes.

### BEST MODE FOR CARRYING OUT THE INVENTION

Hereinafter, the present invention will be described in greater detail with reference to the examples. However, the present invention should not be construed as being limited to these examples. In the examples, all of the base sequence determination does not incorporate a PCR product in a plasmid and the PCR product is directly used as a template. Under standard reaction, isolation, and analysis conditions of the DNA sequencer Model 377 (manufactured by ABI Co., Ltd.), both sequences are decoded.

### EXAMPLE 1: Detection of Activity of Reducing α-Halo-Carbonyl Compound Having α,β-Carbon-Carbon Double Bond

The reduction activity of the compound was detected using α-chloroacrylic acid or α-chloro-α,β-butenoic acid as a substrate by quantitative determination of a reduction product thereof, α-chloropropionic acid or α-chlorobutylic acid with gas chromatography. In addition, 0.4 ml of a reaction solution from which microbial cells were removed by centrifugation or the supernatant of a culture medium was mixed with 0.4 ml of 2N HCl and the resulting mixture was then subjected to a gas chromatographic analysis under the following conditions.
Apparatus: GC-7A (manufactured by Shimadzu Corporation)
Column: Thermon-3000/SHINCARBON A, 2.6 mm x 2.1 m
Carrier gas: nitrogen, 50 ml/min
Detection: FID
Column temperature: 200°C (constant)
Injection: 2 to 10 µl, 260°C
Recording: CHROMATOCODER 12 (SIC)

### EXAMPLE 2

### (1) Cultivation of Pseudomonas sp. SD811 Strain Using Reduction Substrate as Carbon Source

Pseudomonas sp. SD811 strain was incubated in a culture medium containing the following ingredients: α-chloroacrylic acid (2 g/l), yeast extract (0.5 g/l), ammonium sulfate (2 g/l), sodium dihydrogen phosphate (1 g/l), dipotassium hydrogen phosphate (1 g/l), and magnesium sulfate (0.1 g/l).

The medium was prepared as follows.

All of the ingredients mentioned above, except α-chloroacrylic acid and magnesium sulfate, were dissolved in 950 ml of water. The solution obtained was adjusted to a pH of 7.0, and was then poured into a 5-liter flask and sterilized at 121°C for 20 minutes. Subsequently, after the temperature of the medium decreased to about 70°C, a solution prepared by dissolving α-chloroacrylic acid and magnesium sulfate in 50 ml of water was adjusted to a pH of 7.0, sterilized through a sterilization filter, and mixed with the medium prepared above. Without oxygen supply or pH adjustment any more, a 5% seed culture (OD 660 nm = 1.10) was inoculated in the medium and the microbial strain was incubated at 30°C for 12 to 24 hours while being shaken.

### (2) Cultivation of Pseudomonas sp. SD811 Strain Using Reduction Product as Carbon Source

Pseudomonas sp. SD811 strain was incubated in a culture medium containing the following ingredients: L-lactic acid (2 g/l), yeast extract (0.5 g/l), ammonium sulfate (2 g/l), sodium dihydrogen phosphate (1 g/l), dipotassium hydrogen phosphate (1 g/l), and magnesium sulfate (0.1 g/l).

The medium was prepared as follows.

All of the ingredients mentioned above, except L-lactic acid and magnesium sulfate, were dissolved in 950 ml of water. The solution obtained was adjusted to a pH of 7.0, and was then poured into a 5-liter flask and sterilized at 121°C for 20 minutes. Subsequently, after the temperature of the medium decreased to about 70°C, a solution prepared by dissolving L-lactic acid and magnesium sulfate in 50 ml of water was adjusted to a pH of 7.0, sterilized through a sterilization filter, and mixed with the medium prepared above. Without oxygen supply or pH adjustment any more, a 5% seed culture (OD 660 nm = 1.10) was inoculated in the medium and the microbial strain was incubated at 30°C for 12 to 24 hours while being shaken.

### EXAMPLE 3: Cell Suspension Reaction Using α-Chloroacrylic Acid as Substrate

In Example 2, two cultures of Pseudomonas sp. SD811 strain cultivated using two different carbon sources were independently centrifuged to collect the microbial cells. Then, the microbial cells were suspended in 20 ml of a solution (adjusted to a pH of 7.3) containing 0.2% of α-chloroacrylic acid and 100 mM of phosphate buffer (pH 7.3), and the suspension was then reacted at 28°C while being shaken.

From the reaction solution, 0.5 ml was sampled at a specific time and the sample was centrifuged to remove the microbial cells. After that, 0.4 ml of the supernatant from which the microbial cells were removed by centrifugation and 0.1 ml of 6NHC1 were mixed together and then the product was extracted with 0.4 ml of ethyl acetate. The sample extracted was analyzed by the method described in Example 1.

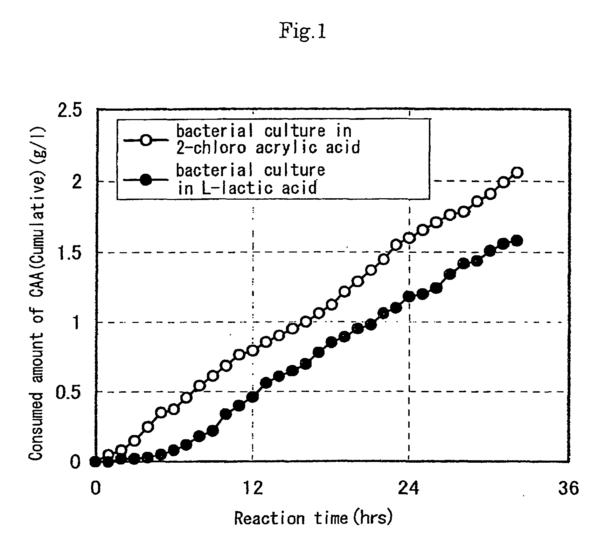

As a result, in the reaction of microbial cells incubated using a reduction substrate, in association with the consumption of α-chloroacrylic acid immediately after the reaction, a peak appeared at the position of α-chloropropionic acid. The reaction rate varied in a nearly linear fashion until the entire substrate was consumed. On the other hand, in the case of the reaction of microbial cells incubatedwith lactic acid as a carbon source, neither the consumption of α-chloroacrylic acid nor the peak of α-chloropropionic acid was detected immediately after the initiation of the reaction. However, after about 5 hours from the initiation, the reduction activity of themicrobial cells gradually increased. Fig. 1 shows the results.

### EXAMPLE 4

### (1) Cultivation of Burkholderia sp. SD816 Strain Using Reduction Substrate as Carbon Source

Burkholderia sp. SD816 strain was incubated in a culture medium containing the following ingredients: α-chloroacrylic acid (2 g/l), yeast extract (0. 5 g/l), ammonium sulfate (2 g/l), sodium dihydrogen phosphate (1 g/l), dipotassium hydrogen phosphate (1 g/l), and magnesium sulfate (0.1 g/l).

The medium was prepared as follows.

All of the ingredients mentioned above, except α-chloroacrylic acid and magnesium sulfate, were dissolved in 950 ml of water. The solution obtained was adjusted to a pH of 7.0, and was then poured into a 5-liter flask and sterilized at 121°C for 20 minutes. Subsequently, after the temperature of the medium decreased to about 70°C, a solution prepared by dissolving α-chloroacrylic acid and magnesium sulfate in 50 ml of water was adjusted to a pH of 7.0, sterilized through a sterilization filter, and mixed with the medium prepared above.

Without oxygen supply or pH adjustment any more, a 5% seed culture (OD 660 nm = 1.10) was inoculated in the medium and the microbial strain was incubated at 30°C for 12 to 24 hours while being shaken.

### (2) Cultivation of Burkholderia sp. SD816 Strain Using Sugar as Carbon Source

Burkholderia sp. SD816 strain was incubated in a culture medium containing the following ingredients: D-glucose (2 g/l), yeast extract (0.5 g/l), ammonium sulfate (2 g/l), sodium dihydrogen phosphate (1 g/l), dipotassium hydrogen phosphate (1 g/l), and magnesium sulfate (0.1 g/l).

The medium was prepared as follows.

All of the ingredients mentioned above, except D-glucose and magnesium sulfate, were dissolved in 950 ml of water. The solution obtained was adjusted to a pH of 7.0, and was then poured into a 5-liter flask and sterilized at 121°C for 20 minutes. Subsequently, after the temperature of the medium decreased to about 70°C, a solution prepared by dissolving D-glucose and magnesium sulfate in 50 ml of water was adjusted to a pH of 7.0, sterilized through a sterilization filter, and mixed with the medium prepared above.

Without oxygen supply or pH adjustment any more, a 5% seed culture (OD 660 nm = 1.10) was inoculated in the medium and the microbial strain was incubated at 30°C for 12 to 24 hours while being shaken.

### EXAMPLE 5: Cell Suspension Reaction Using α-Chloro-α,β-Butenoic Acid as Substrate

In Example 4, two cultures of Burkholderia sp. SD816 strain cultivated using two different carbon sources were independently centrifuged to collect the microbial cells. Then, the microbial cells were suspended in 20 ml of a solution (adjusted to a pH of 7.3) containing 0.2% of α-chloro-α,β-butenoic acid and 100 mM of phosphate buffer (pH 7.3), and the suspension was then reacted at 28°C while being shaken.

From the reaction solution, 0.5 ml was sampled at a specific time and the sample was centrifuged to remove the microbial cells. After that, 0. 4 ml of the supernatant from which the microbial cells were removedby centrifugation and 0.1 ml of 6NHC1 were mixed together and then the product was extracted with 0.4 ml of ethyl acetate. The sample extracted was analyzed by the method described in Example 1.

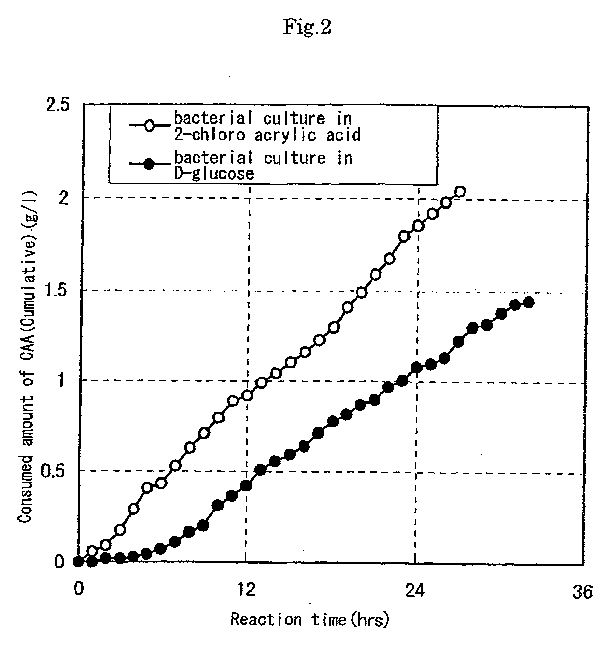

As a result, in the reaction of microbial cells cultivated using a reduction substrate, in association with the consumption of α-chloro-α,β-butenoic acid immediately after the reaction, a peak appeared at the position of α-chlorobutyric acid. The reaction rate varied in a nearly linear fashion until the entire substrate was consumed. On the other hand, in the case of the reaction of microbial cells incubated with lactic acid as a carbon source, neither the consumption of α-chloro-α,β-butenoic acid nor the peak of α-chlorobutyric acid was detected immediately after the initiation of the reaction. However, after about 6 to 10 hours from the initiation, the reduction activity of the microbial cells gradually increased. Fig. 2 shows the results.

### EXAMPLE 6: Analysis of Protein Production Pattern with Two-Dimensional Electrophoresis

### (1) Preparation of Crude Enzyme Extract

The cultures of Pseudomonas sp. SD811 strain, having different reduction activities confirmed in Example 2, were incubated for 18 hours. Then, the microbial cells were collected from each culture by centrifugation. The microbial cells collected were washed with sterilized water, followed by resuspending in 50 mM phosphate buffer (pH 7.5). The microbial cells were broken by a BIOMC 7500 ULTRASONIC PROCESSOR (pulsed, 50 of %duty cycle, about 4.5 of output control) and then unbroken cells and insoluble matters were removed by centrifugation (16,400 x g, 5 min, 4°C).

Similarly, prepared were crude enzyme extracts of the Burkholderia sp. SD816 strain cultures having different reduction activities confirmed in Example 5.

### (2) Primary Electrophoresis: Isoelectric Focusing

A mixture solution was prepared by mixing 1.92 g of urea, 0.53 ml of a 30% acrylamide mixture solution (29.2%(w/v) acrylamide, 0.8% (w/v) N-N' -methylene-bisacrylamide) , and 1. 0 ml of deionized water. After the urea was completely dissolved in the solution, 0.8 ml of 10% Nonidet P-40, 200 µl of Biolight 3/10 Ampholight (BIO-RAD) , 8 µl of 10% ammonium persulfate, and 5.6µl of TEMED were mixed in the solution. Subsequently, the resulting mixture solution was quickly poured into a glass tube (13 mm in length and 2 mm in inner diameter) having a sealed end, and then a 8 M urea solution was layered on the solution, followed by leaving the mixture untouched for 1 to 2 hours to make a solidified gel.

The gel prepared was placed on a semi-micro dry gel electrophoresis apparatus (KS-8110, manufactured by ORIENTAL INSTRUMENTS LTD.), and then a 20 mM sodium hydroxide solution and a 10 mM sulfuric acid solution were poured in upper and lower electrophoresislayers,respectively. Subsequently, the apparatus was pre-activated at 200 V for 15 minutes, 300 V for 15 minutes, and 400 V for 30 minutes.

The sodium hydroxide solution was removed from the upper electrophoresis layer and the upper side of the gel and then a sample solution (prepared by mixing 100 to 300 µg/12.5 µl of protein in solution, 3 µl of 10% Nonidet P-40, 1.5 µl of Biolight 3/10 Ampholight (BIO-RAD), and 1.5 µl of 2-mercaptoethanol) was placed on the upper side of the gel through a syringe. Subsequently, 20 µl of a sample overlay solution (prepared by mixing 0.48 g of urea, 200 µl of 10% Nonidet P-40, 50 µl of Biolight 3/10 Ampholight (BIO-RAD), and 380 ml of deionized water), and a 20 mM sodium hydroxide solution (appropriate amount) were layered on the gel. Then, the upper electrophoresis layer was filled with a 20 mM sodium hydroxide solution, followed by electrophoresing at 400 V for 12 hours and then at 800 V for 1 hour.

After the completion of the primary electrophoresis, the gel was removed from the glass tube and then subjected to shaking in 40 ml of deionized water for 5 minutes at room temperature, followed by shaking in 4 ml of an equilibrating buffer (0.5 ml of 0.5 M Tris-HCl (pH 6.8), 1.6 ml of 10% SDS, 0.05 ml of 0.1% BPB, 2 ml of 2-mercaptoethanol, and 1.65 ml of deionized water were mixed) for 20 minutes at room temperature.

### (3) Secondary Electrophoresis: SDS-PAGE

Sodium dodecyl sulfate-polyacrylamide gel electrophoresis (SDS-PAGE) was performed by a conventional method using a slab gel device (KS-8000 SE type, MARYSOL). More specifically, an equilibrated gel was fixed on the upper end of a 12.5% SDS-PAGE slab gel using 0.5% agarose and then electrophoresis was carried out at a constant current of 25 mA for about 4 hours.

### (4) Detection of Protein: CBB Staining

The detection of proteins in the slab gel after the electrophoresis was performed by ordinary CBB staining. More specifically, the gel was stained for 1 hour in a CBB solution (prepared by dissolving 0.25 g of Coomassie brilliant blue R-250 in 500 ml of methanol, 50 ml of acetic acid, and 450 ml of deionized water), followed by washing with deionized water and then decolorizing for day and night in a decolorizing solution (50 ml of methanol, 70 ml of acetic acid, and 880 ml of deionized water) .

After that, the gel was immersed in a storage solution (23 ml of 87% (w/v) glycerol solution, 150 ml of ethanol, and 327 ml of deionized water) for 3 hours.

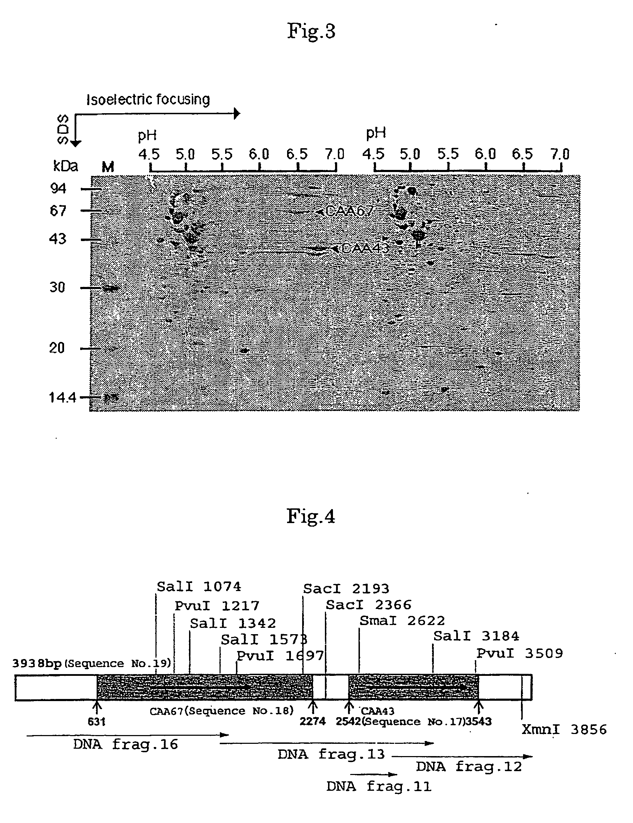

Comparing the two-dimensional electrophoresis separation patterns of crude enzyme extract samples prepared from the microbial cells in L-lactate culture and microbial cells in α-chloroacrylic culture of Pseudomonas sp. SD811 strain, and the microbial cells in D-glucose culture and microbial cells in α-chloroacrylic acid culture of Burkholderia sp. SD816 strain, a number of product proteins specific to the bacterial cells in α-chloroacrylic acid culture were found on the nearly same positions in each set of the respective strains. Fig. 3 shows the results of Pseudomonas sp. SD811 strain.

### EXAMPLE 7(1): Determination of Terminal Sequence

For analyzing the proteins specific to the microbial cells in α-chloroacrylic acid culture, which were found in Example 6, proteins isolated by secondary electrophoresis from the sample of microbial cells in α-chloroacrylic acid culture of Burkholderia sp. SD816 strain were transferred on a PVDF membrane (Immobilon TMTransfermembranes, pore size: 0.45ml, MILLIPORE) usingasemi-dry transfer device (TRANS-BLOT R SD Semi-dry Electrophoretic Transfer Cell (Bio-Rad)).

The transfer was performed according to the standard instructions of the device at a limiting current of 0.8 mA, 13 V, 0.22 to 0.26A for 45 minutes. After the completion of the transfer, the PVDF membrane was stained with the CBB solution. Subsequently, spots corresponding to three different kinds of proteins specifically appeared on the sample of microbial cells in α-chloroacrylic acid culture were cut out and analyzed on a peptide sequencer (Model 491 Procise (Applied Biosystems)). The result showed that one kind of the proteins was a well-known enzyme, dehalogenase (L-DEX), while the remaining two kinds of the proteins had novel peptide sequences represented by SEQ ID. NOS: 1 and 3.

### EXAMPLE 7(2): Determination of Internal Sequence

For acquiring further sequence information, two kinds of novel proteins shown in Example 7 were subjected to in-gel partial digesition using lysylendopeptidase.

After the two-dimensional electrophoresis in Example 6, portions corresponding to two target spots were cut out of the CBB-stained gel. Then,a Tris-buffer containing lysylendopeptidase was added to such a gel section to digest the gel section overnight at 35°C. After that, the reaction solution was subjected to reversed-phase HPLC under the following conditions to isolate fragmented peptides.
Column: TSK gel ODS-120T
Solvent: TFA/Acetonitrile system, gradient elution
Flow rate: 1.0 ml/min
Detection wavelength: 210 nm

From the resulting chromatogram, an appropriate peak was selected and the fraction thereof was analyzed using a peptide sequencer (Model 491 Procise (Applied Biosystems)). As a result, three internal amino acid sequences represented by SEQ ID NOS: 2, 4, and 5 were obtained.

### EXAMPLE 8: Obtaining N-terminal Portion Gene Fragment of CAA43

At first, degenerate primers 1 and 2 were designed on the basis of the N-terminal amino acid sequence of CAA43 and the internal amino acid sequence described in SEQ ID NOS: 1 and 2, respectively.

For the extraction of chromosomal DNA from Burkholderia sp. SD816 strain, QIAGEN genomic-tip 100/G and QIAGEN Genomic DNAbuffer set (each manufactured by QIAGEN) were used.

Using BIO-RAD iCycler (manufactured by BIO-RAD), PCR was carried out under the following conditions.

### [Composition of Reaction Solution]

| | |
|---|---|
| Chromosomal DNA of Burkholderia sp. SD816 | 5 ng |
| Primer 1 (corresponding to SEQ ID NO: 1) | 10 pmol |
| Primer 2 (corresponding to SEQ ID NO: 2) | 10 pmol |
| TaKaRa LATaq | 0.5 unit |
| dNTP mixture (2.5 mM each) | 2.0 µl |
| 10 x LA PCR Buffer II (Mg²⁺ free) | 2.5 µl |
| 25 mM MgCl₂ | 2.5 µl |
| Sterilized distilled water | adjusted to 25 µl |

### [Reaction Cycle]

1 cycle:
   Denaturation (95°C, 4 min),
   Annealing (47.9°C, 1 min), and
   Extension (72°C, 2 min).
2 to 30 cycles:
   Denaturation (95°C, 1 min),
   Annealing (47.9°C, 1 min), and
   Extension (72°C, 2 min).

A DNA fragment (350 bp) , which might encode a part of the CAA43 gene, was obtained by PCR using the chromosomal DNA of Burkholderia sp. SD816 strain as a template. The sequence of the partial fragment was represented by SEQ ID NO: 11.

### Example 9: Obtaining Gene Fragment Encoding C-terminal Region of CAA43

Two downstream primers described in SEQ ID NOS: 8 and 9 were designed according to the base sequence represented by SEQ ID.: 11 obtained in Example 8. The cloning of a gene encoding the C-terminal side of CAA43 was tried using those primers and a TaKaRa LA PCR in vitro Cloning Kit. A reaction or the like was conducted according to the standard instructions attached to the kit. As a result, a DNA fragment (1.3 kb) was obtained by PCR using the chromosomal DNA of Burkholderia sp. SD816 strain treated with XbaI as a template, and was then sequenced. The resulting base sequence was represented by SEQ ID NO: 12 and also a stop codon was identified in this sequence.

### EXAMPLE 10: Obtaining Genes on N-Terminal Region of CAA43 and Upstream Thereof

Aprimer for inverted PCR described in SEQ ID NO: 10 was designed according to the base sequence represented by SEQ ID NO: 11 obtained in Example 8 (see "Basics for Genome Engineering", TOKYO KAGAKU DOJIN CO., LTD. (2002)). This primer was combined with the primer described in SEQ ID NO: 8. Then, the inverted PCR was carried out using the chromosomal DNA of Burkholderia sp. SD816 strain treated with salI as a template under the following conditions.

### [Composition of Reaction Solution]

| | |
|---|---|
| SalI treated product of SD816 strain chromosomal DNA | 200 ng |
| Primer 1 (SEQ ID NO: 8) | 10 pmol |
| Primer 2 (SEQ ID NO: 10) | 10 pmol |
| TaKaRa LATaq | 2.5 units |
| dNTP mixture (2.5 mM each) | 8.0 µl |
| 10 x LA PCR Buffer II (Mg²⁺ free) | 5.0 µl |
| 25 mM MgCl₂ | 5.0 µl |
| Sterilized distilled water | adjusted to 50 µl |

### [Reaction Cycle]

1 cycle:
   Denaturation (94°C, 4.5 min),
   Annealing (55°C, 30 sec), and
   Extension (72°C, 4 min).
2 to 30 cycles:
   Denaturation (94°C,30 sec),
   Annealing (55°C, 30 sec), and
   Extension (72°C, 4 min).

As a result, a DNA fragment of about 1.3 kb was obtained. The base sequence of this fragment was represented by SEQ ID NO: 13. The fragment includes the 0.5 kb amino acid sequence of the N-terminal region of CAA43 and a portion encoding the sequence of CAA67 represented by SEQ ID NO: 4. The inventors found that the fragment includes a 0.8 kb portion which may encode the amino acid sequence of the C-terminal region of CAA67. The coding region of CAA67 resides sequentiallyon the upstreamof the coding region of CAA43. Therefore, the inventors found that both genes forms clusters.

### EXAMPLE 11: Obtaining DNA Fragment Encoding N-Terminal of CAA67

Two primers on the upstream of CAA67 gene described in SEQ ID NOS: 14 and 15 were designed according to the base sequence revealed in Example 10 encoding the internal amino acid sequence of CAA67. The cloning of a gene encoding the N-terminal side CAA67 was tried using those primers and the TaKaRa LA PCR in vitro Cloning Kit. A reaction or the like was conducted according to the standard instructions attached to the kit. As a result, a DNA fragment (1.8 kb) was obtained by PCR using the chromosomal DNA of Burkholderia sp. SD816 strain treated with PstI as a template, and was then sequenced. The resulting base sequence was represented by SEQ ID NO: 12. The inventors confirmed that the DNA fragment was one encoding the internal amino acid sequence of CAA67 described in SEQ ID NOS: 4 and 5 and the DNA fragment encoding the N-terminal amino acid sequence of CAA67 described in SEQ ID NO: 3.

### EXAMPLE 12: Determination of Entire Sequence of Each Gene and Gene Cluster Sequence

FromDNA fragments obtained in Examples 8, 9, and 10, the entire base sequence of CAA43 gene described in SEQ ID NO: 17 was determined using automatic connection-of-nucleic-acid-sequences software (GENETYX-WIN/ATSQ). Similarly, the entire base sequence of CAA67 gene described in SEQ ID NO: 18 was determined using the DNA fragments obtained in Examples 10 and 11. Furthermore, a cluster base sequence described in SEQ ID NO: 19 containing both genes was determined using the DNA fragments obtained in Examples 8 to 11. SEQ ID NOS: 20 and 21 are amino acid sequences corresponding to SEQ ID NOS: 17 and 18, respectively.

### EXAMPLE 13 : Preparation of Gene Fragment Containing CAA43 and CAA67

Primers described in SEQ ID NOS: 22 and 23 were designed according to the base sequence represented by SEQ ID NO: 19 obtained in Example 12. Then, those primers were combined together and subj ected to PCR using the chromosomal DNA of Burkholderia sp. SD816 strain as a template under the following conditions to prepare a 2,913 bp DNA fragment encoding the whole length of the reductase gene.

### [Composition of Reaction Solution] (50 µl in total)

| | |
|---|---|
| SD816 strain chromosomal DNA (5 µg/µl) | 4.0 µl |
| 10 µM primer 1 (SEQ ID NO: 22) | 1.5 µl |
| 10 µM primer 2 (SEQ ID NO: 23) | 1.5 µl |
| TOYOBO KOD-Plus-(1 unit/µl) | 1.0 µl |
| dNTP mixture (2.5 mM each) | 5.0 µl |
| 10 x KOD PCR Buffer (Mg²⁺ free) | 5.0 µl |
| 25 mM MgCl₂ | 2.0 µl |
| Sterilized distilled water | 30 µl |

### [Reaction Cycle]

1 cycle:
   Denaturation (94°C, 2 min),
2 to 30 cycles:
   Denaturation (94°C, 15 sec),
   Annealing (52.3°C, 30 sec), and
   Extension (68°C, 3 min).

### EXAMPLE 14: Construction of CAA43 and CAA67 Expression Systems

The DNA fragment obtained in Example 13 was inserted into the downstream of T7 promoter in the expression vector pET101/D-TOPO, followed by introducing into Escherichia coli BL21(DE3). Ligation between the insert and the vector, transformation, and gene expression were performed using a pET101 Directional TOPO-Expression Kit (Invitrogen).

### EXAMPLE 15: Reduction Reaction Using CAA43 and CAA67 Active Microbial Cells

The microbial cells obtained in Example 14 were incubated in a 5 ml LB culture medium (1% Bacto Tryptone (DIFCO), 0.5% Bacto Yeast Extract (DIFCO), 1% Sodium chloride (Nacalai Tesque), and 100 mg/ml ampicillin) (37°C, 130 rpm, 10 h.). The resulting cells were suspended in 1 ml of 60 mM phosphate buffer (1mM DTT added, pH 7.1). Then, the microbial cells were disrupted by sonication (BRANSON Digital Sonifier) and then centrifuged (15,000 rpm, 4°C, and 10 min). The reduction activity of the supernatant of the cell-disrupted solution was measured according to the method shown in Example 1. At this time, various co-enzymes were added to the reaction solution and the reduction activity thereof was then measured. Consequently, a sufficient reduction activity was observed only when NADPH (reduced nicotinamide adenine dinucleotide phosphate) was added to the reaction solution.

Next, 1/10 volume of NADPH was added to a reaction solution (3 mM 2-CAA, 0. 65 mM NADPH, 60 mM Ammonium acetate buffer (pH 7.1)) and then the decrease of NADPH over time at the time of reacting at 30°C was measured by variations in absorbance at 339 nm in a cell having an optical path length of 0.2 cm. The enzyme level to decrease 1 mmol of NADPH per minute was defined as an enzymatic activity of 1 unit to calculate a specific activity (units/mg). Table 1 shows the 2-CAA reductase activity of the transformant and that of E.coli BL21 (DE3). A significant 2-CAA reduction activity was observed in the transformant.

**Table 1**

| 2-CAA reductase activities of transformant and host | |
|---|---|
| Strain | Specific activity (units/mg) |
| E. coli BL21 (DE3) | 0.06 |
| E. coli BL21 (DE3) pET101/D/67&43 | 0.92 |

### INDUSTRIAL APPLICABILITY

The present invention provide a base sequence encoding a related enzyme having a high catalytic activity useful in producing a corresponding α-substituted-α, β-saturated carbonyl compound from an α-substituted carbonyl compound having an α, β-carbon-carbon double bond by reducing the carbon-carbon double bond using an enzyme produced by a microorganism by a process favored with high profitability, good operability, and excellent processing safety. Furthermore, the present invention provides a reductase and a gene product thereof useful in producing a corresponding highly-purified and optically-active α-substituted-α,β-saturated carbonyl compound, which is useful as chiral building blocks of medical and agricultural chemicals and the like with respect to the α position, from an α-substituted carbonyl compound having an α, β-carbon-carbon double bond prochiral at the α-position by hydrogenating the carbon-carbon double bond.

| Address | Name | Deposit date | Deposit number |
|---|---|---|---|
| Central 6, Higashi 1-chome 1-1, Tsukuba-shi, Ibaraki prefecture, Japan (Postal code number, and 305-8566) | International Patent Organism Depository National Institute of Advanced Industrial Science Technology, an Independent Administrative Institution | 1998/4/2 | FERM BP-6767 |
| | | 1998/4/2 | FERM BP-6768 |
| | | 1998/4/2 | FERM BP-6769 |
| | | 1999/6/28 | FERM BP-6770 |

## Claims

1. A gene including: DNA having a base sequence represented by SEQ ID NO: 19 that encodes a protein having a reduction activity to an α-substituted-α,β-unsaturated carbonyl compound; or DNA that hybridizes with the DNA under stringent conditions.

2. A gene including: DNA having a base sequence represented by SEQ ID NO: 17 that encodes a protein having a reduction activity to an α-substituted-α,β-unsaturated carbonyl compound; or DNA that hybridizes with the DNA under stringent conditions.

3. A gene including: DNA having a base sequence represented by SEQ ID NO: 18 that encodes a protein having a reduction activity to an α-substituted-α,β-unsaturated carbonyl compound; or DNA that hybridizes with the DNA under stringent conditions.

4. A reductase gene for an α-substituted-α,β-unsaturated carbonyl compound, **characterized by** including a DNA sequence encoding an amino acid sequence represented by SEQ ID NO: 20 and an amino acid sequence represented by SEQ ID NO: 21.

5. A gene that encodes a protein having a reduction activity to an α-substituted-α,β-unsaturated carbonyl compound, comprising an amino acid sequence represented by SEQ ID NO: 20 or an amino acid sequence having deletion, substitution, or addition of one or more amino acids.

6. A gene that encodes a protein having a reduction activity to an α-substituted-α,β-unsaturated carbonyl compound, comprising an amino acid sequence represented by SEQ ID NO: 21 or an amino acid sequence having deletion, substitution, or addition of one or more amino acids.

7. A reductase gene for an α-substituted-α,β-unsaturated carbonyl compound according to any one of claims 1 to 6, in which the reductase gene for an α-substituted-α,β-unsaturated carbonyl compound is derived from at least one microorganism selected from the group consisting of the genus Acetobacter, Actinomyces, Acinetobacter, Agrobacterium, Aeromonas, Alcaligenes, Arthrobacter, Azotobacter, Bacillus, Brevibacterium, Burkholderia, Cellulomonas, Corynebacterium, Enterobacter, Enterococcus, Escherichia, Flavobacterium, Gluconobacter, Halobacterium, Halococcus, Klebsiella, Lactobacillus, Microbacterium, Micrococcus, Micropolyspora, Mycobacterium, Nocardia, Pseudomonas, Pseudonocardia, Rhodococcus, Rhodobacter, Serratia, Staphylococcus, Streptococcus, Streptomyces, and Xanthomonas.

8. A reductase gene for an α-substituted-α,β-unsaturated carbonyl compound according to claim 7, in which the reductase gene for an α-substituted-α,β-unsaturated carbonyl compound is derived from a Pseudomonas microorganism.

9. A reductase gene for an α-substituted-α,β-unsaturated carbonyl compound according to claim 7, in which the reductase gene for an α-substituted-α,β-unsaturated carbonyl compound is originated from a Burkholderia microorganism.

10. A reductase gene for an α-substituted-α,β-unsaturated carbonyl compound according to claim 8, in which the Pseudomonas microorganism is Pseudomonas sp. SD810 strain.

11. A reductase gene for an α-substituted-α,β-unsaturated carbonyl compound according to claim 8, in which the Pseudomonas microorganism is Pseudomonas sp. SD811 strain.

12. A reductase gene for an α-substituted-α,β-unsaturated carbonyl compound according to claim 8, in which the Pseudomonas microorganism is Pseudomonas sp. SD812 strain.

13. A reductase gene for an α-substituted-α,β-unsaturated carbonyl compound according to claim 9, in which the Burkholderia microorganism is Burkholderia sp. SD816 strain.

14. A reductase gene for an α-substituted-α,β-unsaturated carbonyl compound according to any one of claims 1 to 13, in which the reductase has a catalytic activity to reduce a carbon-carbon double bond to produce an S-form compound chiral at an α-position.

15. A reductase gene for an α-substituted-α,β-unsaturated carbonyl compound according to any one of claims 1 to 14, in which:
the α-substituted-α,β-unsaturated carbonyl compound is a compound represented by the following general formula (1) 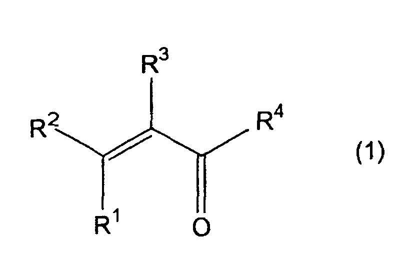 wherein R¹, R², and R³ each independently represent a hydrogen atom, a halogen atom, a linear or branched aliphatic hydrocarbon group having 1 to 6 carbon atoms, a linear or branched alkoxy group having 1 to 6 carbon atoms, a hydroxyl group, a carboxyl group, an aromatic group or a nitrogen-, oxygen-, or sulfur-containing heterocyclic group which may be substituted, and R⁴ represents a hydroxyl group, a linear or branched alkoxy group having 1 to 3 carbon atoms, or a primary, secondary, or tertiary amino group, provided that R³ is not a hydrogen atom; and
a reduced compound is a compound represented by the following general formula (2) 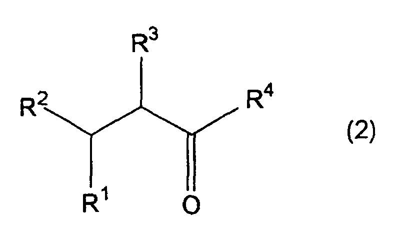 wherein R¹ to R⁴ have the same meanings as those defined above.

16. A reductase gene for an α-substituted-α,β-unsaturated carbonyl compound according to claim 15, in which:
the α-substituted-α,β-unsaturated carbonyl compound is an α-haloacrylic acid represented by the following general formula (1) 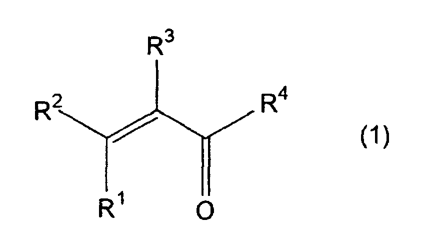 wherein R¹ and R² represent hydrogen atoms, R³ represents a halogen atom, and R⁴ represents a hydroxyl group; and
the reduced compound is an α-halopropionic acid having an S absolute configuration represented by the following general formula (2) 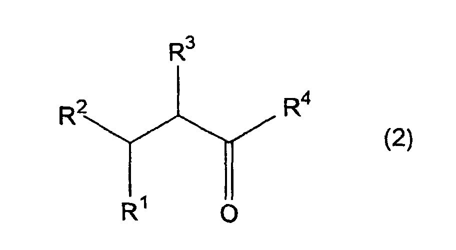 wherein R¹ to R⁴ have the same meanings as those defined above.

17. A reductase gene for an α-substituted-α,β-unsaturated carbonyl compound according to claim 16, in which R³ represents a bromine atom.

18. A reductase gene for an α-substituted-α,β-unsaturated carbonyl compound according to claim 16, in which R³ represents a chlorine atom.

19. A reductase gene for an α-substituted-α,β-unsaturated carbonyl compound according to claim 16, in which R³ represents a fluorine atom.

20. A plasmid, **characterized by** containing a DNA of a reductase gene for an α-substituted-α,β-unsaturated carbonyl compound according to any one of claims 1 to 19.

21. A plasmid, **characterized by** containing a reductase gene for an α-substituted-α,β-unsaturated carbonyl compound according to any one of claims 1 to 19 and a gene for an enzyme functioning with an NADPH as a co-enzyme.

22. A transformant transformed with a plasmid according to claim 20 or 21.

23. A transformant including a product transformed by a plasmid according to claim 20, and a plasmid containing a gene for an enzyme functioning with an NADPH as a co-enzyme.

24. A protein having an activity to reduce an α-substituted-α,β-unsaturated carbonyl compound wherein the proten is an expression product of a reductase gene for the α-substituted-α,β-unsaturated carbonyl compound according to any one of claims 1 to 19, or a protein having deletion, substitution, or addition of one or more amino acids thereof and having a reduction activity to an α-substituted-α,β-unsaturated carbonyl compound.

25. A protein having an activity to reduce an α-substituted-α,β-unsaturated carbonyl compound containing an amino acid sequence represented by SEQ ID NO: 20 or an amino acid sequence having deletion, substitution, or addition of one or more amino acids in the said amino acid sequence.

26. A protein having an activity to reduce an α-substituted-α,β-unsaturated carbonyl compound containing an amino acid sequence represented by SEQ ID NO: 21 or an amino acid sequence having deletion, substitution, or addition of one or more amino acids in the said amino acid sequence.

27. A method of producing a gene that encodes a protein having an activity to reduce an α-substituted-α,β-unsaturated carbonyl compound, comprising using a pair of primers prepared by combining a base sequence selected from base sequences located upstream of abase at position 631 and a base sequence selected frombase sequences located downstream of a base at position 3,543 in the base sequence represented by SEQ ID NO: 19, where both the base sequences extend in opposite directions to each other.

28. A method of producing a gene that encodes a protein having an activity to reduce an α-substituted-α,β-unsaturated carbonyl compound, comprising using a pair of primers prepared by combining a base sequence selected from base sequences located upstream of abase atposition 631 and a base sequence selected frombase sequences located downstream of a base at position 2,274 in base sequences represented by SEQ ID NO: 19, where both base sequences extend in opposite directions to each other.

29. A method of producing a gene that encodes a protein having an activity to reduce an α-substituted-α,β-unsaturated carbonyl compound, comprising using a pair of primers prepared by combining a base sequence selected from base sequences located upstream of a base at position 2,547 and a base sequence selected from base sequences located downstream of a base at position 3,543 in base sequences represented by SEQ ID NO: 19, where both base sequences extend in opposite directions so as to be reversed strands with respect to each other.

30. Amethod of reducing an α-substituted-α,β-unsaturated carbonyl compound, comprising:
using a culture and/or treated product of a transformant according to claim 22 or 23.
